# EUROPÄISCHE PATENTSCHRIFT

(11) **EP 3 492 104 B1**
(45) Veröffentlichungstag und Bekanntmachung des Hinweises auf die Patenterteilung: **15.02.2023**
(21) Anmeldenummer: 18209950.7
(22) Anmeldetag: 22.06.2012
(51) Int. Cl.: A61K 41/00, A61K 31/00, A61K 31/525

(54) **VERWENDUNG VON 10H-BENZO[G]PTERIDIN-2,4-DION-DERIVATEN**
USE OF 10H-BENZO [G] PTERIDIN-2,4-DION DERIVATIVES
UTILISATION DE DÉRIVÉS DE 10H-BENZO[G]PTERIDINE-2,4-DIONE

(30) Priorität: 22.06.2011 DE 102011105660
(43) Veröffentlichungstag der Anmeldung: 05.06.2019
(62) Teilanmeldung aus: 12729136.7
(73) Patentinhaber: TriOptoTec GmbH, 93053 Regensburg (DE)
(72) Erfinder: SPÄTH, Andreas, Dr., 93055 Regensburg (DE); MAISCH, Tim, Prof. Dr., 90425 Nürnberg (DE)
(74) Vertreter: Louis Pöhlau Lohrentz

(56) Entgegenhaltungen:
- WO-A1-2011/008247
- WO-A2-00/04930
- WO-A2-2011/126576
- WO-A2-2011/126576
- VINCENZO VERDOLINO ET AL: "Calculation of p K a Values of Nucleobases and the Guanine Oxidation Products Guanidinohydantoin and Spiroiminodihydantoin using Density Functional Theory and a Polarizable Continuum Model", THE JOURNAL OF PHYSICAL CHEMISTRY B, Bd. 112, Nr. 51, 25. Dezember 2008 (2008-12-25), Seiten 16860-16873, XP55120801, ISSN: 1520-6106, DOI: 10.1021/jp8068877
- É A RUDZIT ET AL: "Allo- and isoalloxazines LI. Synthesis and antimicrobial activity of allo- and isoalloxazines", PHARMACEUTICAL CHEMISTRY JOU, SPRINGER NEW YORK LLC, US, Bd. 12, Nr. 1, 1. Januar 1978 (1978-01-01) , Seiten 49-54, XP008153978, ISSN: 0091-150X, DOI: 10.1007/BF01188702
- A. JOSHI ET AL: "Recent Developments in Antimalarial Drug Discovery", ANTI-INFECTIVE AGENTS IN MEDICINAL CHEMISTRY, Bd. 5, Nr. 1, 1. Januar 2006 (2006-01-01), Seiten 105-122, XP055574391, NL ISSN: 1871-5214, DOI: 10.2174/187152106774755626
- RAJVIR MALIK ET AL: "Photodynamic therapy - A strategic review", INDIAN JOURNAL OF DENTAL RESEARCH, Bd. 21, Nr. 2, 1. Januar 2010 (2010-01-01) , Seite 285, XP055574208, IN ISSN: 0970-9290, DOI: 10.4103/0970-9290.66659
- CHRISTINA K. REMUCAL ET AL: "Photosensitized Amino Acid Degradation in the Presence of Riboflavin and Its Derivatives", ENVIRONMENTAL SCIENCE & TECHNOLOGY, Bd. 45, Nr. 12, 15. Juni 2011 (2011-06-15) , Seiten 5230-5237, XP055034035, ISSN: 0013-936X, DOI: 10.1021/es200411a

## Beschreibung

Die vorliegende Erfindung betrifft die Verwendung von 10H-Benzo[g]pteridin-2,4-dion-Derivaten.

Das aktive oder passive Eindringen, Anhaften und Vermehren von Krankheitserregern in einen Wirt wird als Infektion bezeichnet. Quellen für infektiöse Partikel treten überall auf. So wird beispielsweise der menschliche Körper von einer großen Anzahl von Mikroorganismen besiedelt, die im Regelfall durch den normalen Metabolismus und ein intaktes Immunsystem unter Kontrolle gehalten werden. Allerdings kann es, beispielsweise bei einer Schwächung des Immunsystems, zu einer starken Vermehrung der Krankheiterreger kommen und je nach Art des Erregers zu unterschiedlichen Krankheitssymptomen. Die Medizin hält für viele erregerbedingte Krankheiten spezifische Gegenmittel bereit, beispielsweise Antibiotika gegen Bakterien oder Antimikotika gegen Pilze oder Virustatika gegen Viren. Allerdings ist bei der Verwendung dieser Gegenmittel vermehrt das Auftreten von resistenten Krankheitserregern zu beobachten, die teilweise gleichzeitig gegen mehrere Gegenmittel Resistenzen aufweisen. Durch das Auftreten dieser resistenten oder multiresistenten Krankheitserreger hat sich die Therapie von Infektionserkrankungen zunehmend erschwert. Die klinische Konsequenz der Resistenz zeigt sich durch ein Versagen der Behandlung, vor allem bei immunsupprimierten Patienten.

Neue Ansatzpunkte zur Bekämpfung von resistenten bzw. multiresistenten Krankheitskeimen sind daher einerseits die Suche nach neuen Gegenmitteln, beispielsweise Antibiotika oder Antimikotika, und andererseits die Suche nach alternativen Inaktivierungsmöglichkeiten.

Als alternatives Verfahren hat sich die photodynamische Inaktivierung von Mikroorganismen bewährt. Bei der photodynamischen Inaktivierung von Mikroorganismen spielen zwei verschiedene photooxidative Prozesse eine entscheidende Rolle. Voraussetzung für den Ablauf einer photooxidativen Inaktivierung ist einerseits das Vorhandensein einer ausreichenden Menge Sauerstoff und andererseits die Lokalisierung eines so genannten Photosensibilisators, welcher durch Licht einer entsprechenden Wellenlänge angeregt wird. Der angeregte Photosensibilisator kann die Bildung von reaktiven Sauerstoffspezies (ROS) bewirken, wobei einerseits Radikale, beispielsweise Superoxidanionen, Wasserstoffperoxid oder Hydroxylradikale, und/oder andererseits angeregter molekularer Sauerstoff, beispielsweise Singulett-Sauerstoff, gebildet werden können.

Bei beiden Reaktionen steht die Photooxidation von spezifischen Biomolekülen, die sich in direkter Nachbarschaft zu den reaktiven Sauerstoffspezies (ROS) befindet, im Vordergrund. Dabei findet insbesondere eine Oxidation von Lipiden und Proteinen statt, die beispielsweise als Bestandteile der Zellmembran von Mikroorganismen vorkommen. Durch die Zerstörung der Zellmembran wiederum kommt es zur Inaktivierung der betreffenden Mikroorganismen. Für Viren und Pilze wird ein ähnlicher Eliminationsprozess angenommen.

Beispielsweise werden durch Singulett-Sauerstoff alle Moleküle angegriffen. Besonders anfällig für eine Schädigung sind allerdings ungesättigte Fettsäuren in den Membranen von Bakterien. Gesunde, körpereigene Zellen verfügen über eine zelluläre Abwehr gegen Angriffe von freien Radikalen, die so genannten Katalasen oder Superoxiddismutasen. Daher können gesunde, körpereigene Zellen eine Schädigung durch reaktive Sauerstoffspezies (ROS), beispielsweise Radikale oder Singulett-Sauerstoff, entgegenwirken.

Aus dem Stand der Technik sind zahlreiche Photosensibilisatoren bekannt, die beispielsweise aus der Gruppe der Porphyrine und deren Derivate oder Phthalocyanine und deren Derivate oder Fullerene und deren Derivate oder Derivate der Phenothiazininium-Struktur, wie beispielsweise Methylenblau oder Toluidinblau, oder Vertreter der Phenoxazinium-Reihe, wie beispielsweise Nile blue, stammen. Die Photodynamik von Methylenblau bzw. Toluidinblau gegenüber Bakterien wird beispielsweise bereits in der Zahnheilkunde eingesetzt.

Bei den aus dem Stand der Technik bekannten Photosensibilisatoren handelt es sich zumeist um Substanzen mit einer relativ komplexen Molekülstruktur und daher aufwendigen Herstellungsverfahren.

Es ist bekannt, dass 10-Methyl-10H-benzo[g]pteridine-2,4-dion-Derivate, Riboflavin und Tetraacetylriboflavin hohe Ausbeuten an Singulett-Sauerstoff haben, wobei jedoch ihre Affinität zu Mikroorganismen gering ist. Es ist weiterhin bekannt, dass Singulett-Sauerstoff lediglich über eine geringe Entfernung diffundieren kann, bevor er reagiert oder abgebaut wird. Daher ist die Inaktivierung von Mikroorganismen durch 10-Methyl-10H-benzo[g]pteridine-2,4-dion-Derivate, Riboflavin oder Tetraacetylriboflavin unzureichend.

Weiterhin sind aus der WO 2010/019208 A1 und der WO 2011/008247 A1 zahlreiche Flavin-, Roseoflavin- und Riboflavin-Derivate bekannt, die an Flavinmononucleotid (FMN) Riboswiches binden können. Riboswiches sind RNA-Elemente in untranslatierten Regionen der mRNA von Prokaryoten, Pilzen und Pflanzen, die niedermolekulare Metabolite, beispielsweise FMN, binden und daraufhin die Genexpression regulieren. Beispielsweise wird nach Bindung von FMN an FMN-Riboswiches von Prokaryoten die Expression von Enzymen, die für die Riboflavinund FMN-Biosynthese verantwortlich sind, reprimiert, wodurch die Riboflavin- und FMN-Biosynthese zum Erliegen kommt. Riboflavin nimmt im Stoffwechsel eine zentrale Rolle ein, da es als Vorstufe für Flavin-Koenzyme dient. Daher führt eine unterdrückte Riboflavin- und FMN-Biosynthese zu einer reduzierten Überlebensfähigkeit. Auch RAJVIR MALIK ET AL: "Photodynamic therapy - A strategic review",INDIAN JOURNAL OF DENTAL RESEARCH, Bd. 21, Nr. 2, 1. Januar 2010 (2010-01-01), Seite 285 und CHRISTINA K. REMUCAL ET AL: "Photosensitized Amino Acid Degradation in the Presence of Riboflavin and Its Derivatives",ENVIRONMENTAL SCIENCE & TECHNOLOGY, Bd. 45, Nr. 12, 15. Juni 2011 , Seiten 5230-5237 und WO0004930 sind zu erwähnen.

Allerdings kann es bei dieser Form der Bekämpfung von pathogenen Mikroorganismen ebenfalls zum Auftreten von Resistenzen kommen, die beispielsweise durch Mutationen der entsprechenden RNA-Elemente entstehen können.

Aufgabe der vorliegenden Erfindung ist es Mikroorganismen effizienter inaktivieren.

Die Aufgabe der der vorliegenden Erfindung wird gelöst durch die Verwendung einer Verbindung mit der Formel (1): 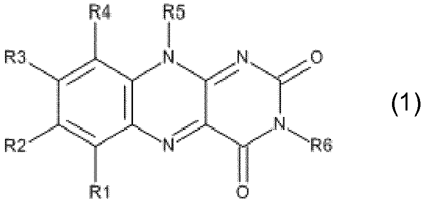 zur Anwendung gemäss Anspruch 1,
wobei nur 1 Rest R1, R2, R3 oder R4 ein organischer Rest der allgemeinen Formel (2), (3) oder (4):

-(C(D)(E))ₕ-X, (2)

-(C(D)(E))ₖ-Aryl-(C(D)(E))ₗ-X, (3)

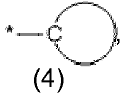

ist,
wobei h eine ganze Zahl von 1 bis 20 und k und I jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeutet und wobei X ein organischer Rest ist, der a) nur ein neutrales, protonierbares Stickstoffatom enthält oder b) nur ein positiv geladenes Stickstoffatom, das vorzugsweise kein quartäres Stickstoffatom ist, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
und wobei der Rest mit der Formel (4) ein Heteroaromat, der über ein C-Atom des Heteroaromaten an den Isoalloxazinring gebunden ist und der a) nur ein neutrales, protonierbares Stickstoffatom oder b) nur ein positiv geladenes Stickstoffatom enthält bedeutet und
wobei die Reste R1, R2, R3 oder R4, die nicht ein organischer Rest der allgemeinen Formel (2), (3) oder (4) sind, jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten und
wobei jeder der Reste R5 oder R6 jeweils unabhängig voneinander gleich oder verschieden ist und Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet
oder
wobei nur 1 Rest R5 oder R6 ein organischer Rest der allgemeinen Formel (2), (3) oder (4):

   -(C(D)(E))ₕ-X, (2)

   -(C(D)(E))ₖ-Aryl-(C(D)(E))ₗ-X, (3)

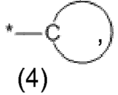

   ist,
wobei h eine ganze Zahl von 1 bis 20 und k und I jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeutet und wobei X ein organischer Rest ist, der a) nur ein neutrales, protonierbares Stickstoffatom enthält oder b) nur ein positiv geladenes Stickstoffatom, das vorzugsweise kein quartäres Stickstoffatom ist, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
und wobei der Rest mit der Formel (4) ein Heteroaromat, der über ein C-Atom des Heteroaromaten an den Isoalloxazinring gebunden ist und der a) nur ein neutrales, protonierbares Stickstoffatom oder b) nur ein positiv geladenes Stickstoffatom enthält, bedeutet und
wobei der Rest R5 oder R6, der nicht ein organischer Rest der allgemeinen Formel (2), (3) oder (4) ist, Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet und
wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Bei der erfindungsgemäß verwendeten Verbindung mit der Formel (1) handelt es sich um ein 10H-Benzo[g]pteridin-2,4-dion- oder Flavin-Derivat, das im Folgenden auch so bezeichnet wird.

Als Gegenion zum positiv geladenen Stickstoffatom kann jedes geeignete Anion verwendet werden. Vorzugsweise werden als Gegenion zum positiv geladenen Stickstoffatom Anionen verwendet, die die Bereitstellung eines pharmakologisch verträglichen Salzes ermöglichen.

Bei einer bevorzugten Ausführungsform der vorliegenden Erfindung ist X in der Verbindung mit der Formel (1) ein organischer Rest mit nur einem positiven Stickstoffatom, das als Gegenion Fluorid, Chlorid, Bromid, Iodid, Sulfat, Hydrogensulfat, Phosphat, Dihydrogenphosphat, Hydrogenphosphat, Tosylat, Mesylat, Formiat, Acetat, Oxalat, Benzoat, Citrat und/oder Mischungen davon aufweist.

Bei einer weiter bevorzugten Ausführungsform ist der Rest X ein organischer Rest, der nur ein (Zahl= 1) basisches, vorzugsweise neutral, protonierbares oder positiv geladenes, Stickstoffatom enthält und keine quartären Stickstoffatome.

Weitere bevorzugte Ausführungsformen der vorliegenden Erfindung sind in den abhängigen Ansprüchen beschrieben.

Als "Photosensibilisator" werden erfindungsgemäß Verbindungen verstanden, die elektromagnetische Strahlung, vorzugsweise sichtbares Licht, UV-Licht und/oder Infrarotlicht, absorbieren und sodann reaktive Sauerstoffspezies (ROS), vorzugsweise freie Radikale und/oder Singulett-Sauerstoff, aus Triplett-Sauerstoff erzeugen.

Unter dem Begriff "photodynamische Therapie" wird erfindungsgemäß die lichtinduzierte Inaktivierung von Zellen oder Mikroorganismen verstanden.

Unter dem Begriff "Inaktivierung" wird erfindungsgemäß die Reduzierung der Lebensfähigkeit oder die Zerstörung eines Mikroorganismus, vorzugsweise dessen Zerstörung, verstanden. Eine lichtinduzierte Inaktivierung kann beispielsweise durch Verringerung der Anzahl von Mikroorganismen nach Bestrahlung einer definierten Ausgangsmenge dieser Mikroorganismen in Gegenwart wenigstens einer erfindungsgemäß verwendeten Verbindung mit der Formel (1) bestimmt werden.

Erfindungsgemäß wird unter einer Reduzierung der Lebensfähigkeit verstanden, dass die Anzahl der Mikroorganismen um wenigstens 99,0 %, vorzugsweise um wenigstens 99,9 %, weiter bevorzugt um wenigstens 99,99 %, weiter bevorzugt um wenigstens 99,999 %, noch weiter bevorzugt um wenigstens 99,9999 %, verringert wird. Äußerst bevorzugt wird die Anzahl der Mikroorganismen um mehr als 99,9 bis 100 %, vorzugsweise um mehr als 99,99 bis 100 %, verringert wird.

Vorzugsweise wird die Reduzierung der Anzahl der Mikroorganismen gemäß Boyce, J.M. und Pittet, D. ("Guidelines for hand hygiene in healthcare settings. Recommendations of the Healthcare Infection Control Practices Advisory Committee and the HIPAC/SHEA/APIC/IDSA Hand Hygiene Task Force", Am.J.Infect.Control 30 (8), 2002, Seite 1 - 46) als log₁₀-Reduktionsfaktor angegeben.

Erfindungsgemäß wird unter dem Begriff "log₁₀-Reduktionsfaktor" die Differenz zwischen dem dekadischen Logarithmus der Anzahl der Mikroorganismen vor und dem dekadischen Logarithmus der Anzahl der Mikroorganismen nach einer Bestrahlung dieser Mikroorganismen mit elektromagnetischer Strahlung in Gegenwart wenigstens einer erfindungsgemäß verwendeten Verbindung mit der Formel (1) verstanden.

Geeignete Methoden zur Bestimmung des log₁₀-Reduktionsfaktors sind beispielsweise in der DIN EN 14885:2007-01 "Chemische Desinfektionsmittel und Antiseptika - Anwendung Europäischer Normen für chemische Desinfektionsmittel und Antiseptika" oder in Rabenau, H.F. und Schwebke, I. ("Leitlinie der Deutschen Vereinigung zur Bekämpfung der Viruskrankheiten (DVV) e.V. und des Robert Koch-Instituts (RKI) zur Prüfung von chemischen Desinfektionsmitteln auf Wirksamkeit gegen Viren in der Humanmedizin" Bundesgesundheitsblatt, Gesundheitsforschung, Gesundheitschutz 51(8), (2008), Seiten 937 - 945) beschrieben.

Vorzugsweise beträgt der log₁₀-Reduktionsfaktor nach einer Bestrahlung von Mikroorganismen mit elektromagnetischer Strahlung in Gegenwart wenigstens einer erfindungsgemäß verwendeten Verbindung mit der Formel (1) mindestens 2 log₁₀, vorzugsweise mindestens 3 log₁₀, weiter bevorzugt mindestens 4 log₁₀, weiter bevorzugt mindestens 4,5 log₁₀, weiter bevorzugt mindestens 5 log₁₀, weiter bevorzugt mindestens 6 log₁₀, noch weiter bevorzugt mindestens 7 log₁₀, noch weiter bevorzugt mindestens 7,5 log₁₀.

Beispielsweise bedeutet eine Reduktion der Anzahl der Mikroorganismen nach einer Bestrahlung dieser Mikroorganismen mit elektromagnetischer Strahlung in Gegenwart wenigstens einer erfindungsgemäß verwendeten Verbindungen mit der Formel (1) um 2 Zehnerpotenzen, bezogen auf die Ausgangsmenge dieser Mikroorganismen, einen log₁₀-Reduktionsfaktor von 2 log₁₀.

Weiter bevorzugt wird die Anzahl der Mikroorganismen nach einer Bestrahlung dieser Mikroorganismen mit elektromagnetischer Strahlung in Gegenwart wenigstens einer erfindungsgemäß verwendeten Verbindung mit der Formel (1) um mindestens 1 Zehnerpotenz, weiter bevorzugt um mindestens 2 Zehnerpotenzen, vorzugsweise um mindestens 4 Zehnerpotenzen, weiter bevorzugt um mindestens 5 Zehnerpotenzen, weiter bevorzugt um mindestens 6 Zehnerpotenzen, noch weiter bevorzugt um mindestens 7 Zehnerpotenzen, jeweils bezogen auf die Ausgangsmenge dieser Mikroorganismen, verringert.

Unter dem Begriff "Mikroorganismen" werden im Sinne der Erfindung insbesondere Viren, Archäeen, prokaryote Mikroorganismen, wie Bakterien und Bakteriensporen, und eukaryote Mikroorganismen, wie Pilze, Protozoen, Pilzsporen, einzellige Algen, verstanden. Die Mikroorganismen können dabei einzellig oder mehrzellig, beispielsweise als Pilzmycel, auftreten.

Ein erfindungsgemäß als Photosensibilisator bei der Inaktivierung von Mikroorganismen verwendetes 10H-Benzo[g]pteridin-2,4-dion-Derivat weist die Formel (1) auf 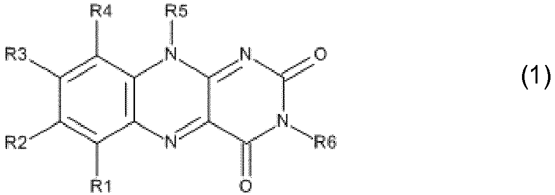 wobei entweder
A) nur 1 Rest R1, R2, R3 oder R4 ein organischer Rest der allgemeinen Formel (2), (3) oder (4):

   -(C(D)(E))ₕ-X, (2)

   -(C(D)(E))ₖ-Aryl-(C(D)(E))ₗ-X, (3)

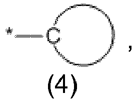

   ist,
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k und I jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeutet und wobei X ein organischer Rest ist, der a) nur ein neutrales, protonierbares Stickstoffatom enthält oder b) nur ein positiv geladenes Stickstoffatom enthält, das vorzugsweise kein quartäres Stickstoffatom ist, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   und wobei der Rest mit der Formel (4) ein Heteroaromat, der über ein C-Atom des Heteroaromaten an den Isoalloxazinring gebunden ist und der a) nur ein neutrales, protonierbares Stickstoffatom und/oder b) nur ein positiv geladenes Stickstoffatom enthält, bedeutet
   oder
B) nur 1 Rest R5 oder R6 ein organischer Rest der allgemeinen Formel (2), (3) oder (4):

   -(C(D)(E))ₕ-X, (2)

   -(C(D)(E))ₖ-Aryl-(C(D)(E))ₗ-X, (3)

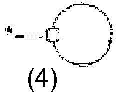

   ist,
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k und l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, ist und wobei X ein organischer Rest ist, der a) nur ein neutrales, protonierbares Stickstoffatom enthält oder b) nur ein positiv geladenes Stickstoffatom, das vorzugsweise kein quartäres Stickstoffatom ist, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   und wobei der Rest mit der Formel (4) ein Heteroaromat, der über ein C-Atom des Heteroaromaten an den Isoalloxazinring gebunden ist und der a) nur ein neutrales, protonierbares Stickstoffatom oder b) nur ein positiv geladenes Stickstoffatom enthält, bedeutet.

Bei einer weiter bevorzugten Ausführungsform des erfindungsgemäß als Photosensibilisator bei der Inaktivierung von Mikroorganismen verwendeten 10H-Benzo[g]pteridin-2,4-dion-Derivats der Formel (1) sind D und E jeweils Wasserstoff.

Bei einer bevorzugten Ausführungsform weist das erfindungsgemäß verwendete 10H-Benzo[g]pteridin-2,4-dion-Derivat der Formel (1) kein neutrales, protonierbares Stickstoffatom, das direkt an den Isoalloxazinring gebunden ist, beispielsweise als Amino-Rest, Methylamino-Rest oder Dimethylamino-Rest, und kein positiv geladenes Stickstoffatom, das direkt an den Isoalloxazinring gebunden ist, beispielsweise als Pyridin-1-ium-1-yl-Rest oder Trimethylammonio-Rest, auf.

Bei einer weiter bevorzugten Ausführungsform enthält das erfindungsgemäß verwendete 10H-Benzo[g]pteridin-2,4-dion-Derivats der Formel (1) keine weiteren basischen, vorzugsweise neutralen, protonierbaren und/oder positiv geladenen, Stickstoffatome und keine quartären Stickstoffatome.

Bei einer weiteren bevorzugten Ausführungsform des erfindungsgemäß als Photosensibilisator bei der Inaktivierung von Mikroorganismen verwendeten 10H-Benzo[g]pteridin-2,4-dion-Derivats der Formel (1) ist entweder
A) nur 1 Rest R1, R2, R3 oder R4 ein organischer Rest der allgemeinen Formel (2), (3) oder (4),
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest ist, der a) nur ein neutrales, protonierbares Stickstoffatom enthält oder b) nur ein positiv geladenes Stickstoffatom, das vorzugsweise kein quartäres Stickstoffatom ist, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   und wobei der Rest mit der Formel (4) ein Heteroaromat, der über ein C-Atom des Heteroaromaten an den Isoalloxazinring gebunden ist und der a) nur ein neutrales, protonierbares Stickstoffatom oder b) nur ein positiv geladenes Stickstoffatom enthält bedeutet und
   wobei die Reste R1, R2, R3 oder R4, die nicht ein organischer Rest der allgemeinen Formel (2), (3) oder (4) sind, jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten und
   wobei der Reste R5 ein nichtcyclischer Polyolrest der allgemeinen Formel -CH₂(CH(OH))_{g}CH₂OH ist oder ein Ether, Ester oder Acetal davon, wobei g eine ganze Zahl von 1 bis 10, vorzugsweise 1 bis 4, bedeutet und
   wobei R6 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet
   oder
B) nur der Rest R6 ein organischer Rest der allgemeinen Formel (2), (3) oder (4),
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest ist, der a) nur ein neutrales, protonierbares Stickstoffatom enthält oder b) nur ein positiv geladenes Stickstoffatom, das vorzugsweise kein quartäres Stickstoffatom ist, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   und wobei der Rest mit der Formel (4) ein Heteroaromat, der über ein C-Atom des Heteroaromaten an den Isoalloxazinring gebunden ist und der a) wenigstens ein neutrales, protonierbares Stickstoffatom oder b) wenigstens ein positiv geladenes Stickstoffatom enthält, bedeutet und
   wobei der Reste R5 ein nichtcyclischer Polyolrest der allgemeinen Formel -CH₂(CH(OH))_{g}CH₂OH ist oder ein Ether, Ester oder Acetal davon, wobei g eine ganze Zahl von 1 bis 10, vorzugsweise 1 bis 4, bedeutet und
   wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Weiter bevorzugt ist der Reste R5 ein nichtcyclischer Polyolrest, der aus der Gruppe, die aus Arabityl, Ribityl, Xylityl, Erythrityl, Threityl, Lactityl, Mannityl und Sorbityl, weiter bevorzugt D-Ribityl und D-Arabityl, besteht, ausgewählt wird oder ein Ether, Ester oder Acetal davon.

Bei Variante A) des erfindungsgemäß als Photosensibilisator bei der Inaktivierung von Mikroorganismen verwendeten 10H-Benzo[g]pteridin-2,4-dion-Derivats der Formel (1) ist nur 1 Rest R1, R2, R3 oder R4 ein organischer Rest der allgemeinen Formel (2), (3) oder (4), wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, X ein organischer Rest ist, der a) nur ein neutrales, protonierbares Stickstoffatom enthält oder b) nur ein positiv geladenes Stickstoffatom, das vorzugsweise kein quartäres Stickstoffatom ist, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
und wobei der Rest mit der Formel (4) ein Heteroaromat, der über ein C-Atom des Heteroaromaten an den Isoalloxazinring gebunden ist und der a) nur ein neutrales, protonierbares Stickstoffatom oder b) nur ein positiv geladenes Stickstoffatom enthält, bedeutet,
wobei die Reste R1, R2, R3 oder R4, die ein organischer Rest der allgemeinen Formel (2), (3) oder (4) sind, jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 2 bis 20 C-Atomen, O-Alkenyl mit 1 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten und
wobei jeder der Reste R5 oder R6 jeweils unabhängig voneinander gleich oder verschieden ist und Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet.

Bei Variante B) des erfindungsgemäß als Photosensibilisator bei der Inaktivierung von Mikroorganismen verwendeten 10H-Benzo[g]pteridin-2,4-dion-Derivats der Formel (1) ist nur 1 Rest R5 oder R6 ein organischer Rest der allgemeinen Formel (2), (3) oder (4), wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten, X ein organischer Rest ist, der a) nur ein neutrales, protonierbares Stickstoffatom enthält und/oder b) nur ein positiv geladenes Stickstoffatom, das vorzugsweise kein quartäres Stickstoffatom ist, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet und
wobei der Rest mit der Formel (4) ein Heteroaromat, der über ein C-Atom des Heteroaromaten an den Isoalloxazinring gebunden ist und der a) nur ein neutrales, protonierbares Stickstoffatom und/oder b) nur ein positiv geladenes Stickstoffatom enthält, bedeutet,
wobei der Rest R5 oder R6, der nicht ein organischer Rest der allgemeinen Formel (2), (3) oder (4) ist, Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet und
wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 1 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Bei einer weiteren bevorzugten Ausführungsform des erfindungsgemäß als Photosensibilisator bei der Inaktivierung von Mikroorganismen verwendeten 10H-Benzo[g]pteridin-2,4-dion-Derivats der Formel (1) ist entweder
A) nur 1 Rest R1, R2, R3 oder R4 ein organischer Rest der allgemeinen Formel (2) oder (3):

   -(C(D)(E))ₕ-X, (2)

   -(C(D)(E))ₖ-Aryl-(C(D)(E))ₗ-X, (3)

   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k und l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest ist, der a) nur ein neutrales, protonierbares Stickstoffatom enthält oder b) nur ein positiv geladenes Stickstoffatom enthält, das vorzugsweise kein quartäres Stickstoffatom ist, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   oder
B) nur 1 Rest R5 oder R6 ein organischer Rest der allgemeinen Formel (2) oder (3):

   -(C(D)(E))ₕ-X, (2)

   -(C(D)(E))ₖ-Aryl-(C(D)(E))ₗ-X, (3)

   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k und I jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest ist, der a) nur ein neutrales, protonierbares Stickstoffatom enthält oder b) nur ein positiv geladenes Stickstoffatom, das vorzugsweise kein quartäres Stickstoffatom ist, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet.

Bei einer weiteren bevorzugten Ausführungsform des erfindungsgemäß als Photosensibilisator bei der Inaktivierung von Mikroorganismen verwendeten 10H-Benzo[g]pteridin-2,4-dion-Derivats der Formel (1) ist entweder
A) nur 1 Rest R1, R2, R3 oder R4 ein organischer Rest der allgemeinen Formel (2) oder (3),
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest ist, der a) nur ein neutrales, protonierbares Stickstoffatom enthält oder b) nur ein positiv geladenes Stickstoffatom, das vorzugsweise kein quartäres Stickstoffatom ist, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   wobei die Reste R1, R2, R3 oder R4, die nicht ein organischer Rest der allgemeinen Formel (2) oder (3) sind, jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten und
   wobei der Reste R5 ein nichtcyclischer Polyolrest der allgemeinen Formel -CH₂(CH(OH))_{g}CH₂OH ist oder ein Ether, Ester oder Acetal davon, wobei g eine ganze Zahl von 1 bis 10, vorzugsweise 1 bis 4, bedeutet und
   wobei R6 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet
   oder
B) nur der Rest R6 ein organischer Rest der allgemeinen Formel (2)oder (3),
   wobei h eine ganze Zahl von 1 bis 20, vorzugsweise von 1 bis 19, vorzugsweise von 1 bis 17, weiter bevorzugt von 1 bis 13, weiter bevorzugt von 1 bis 9, weiter bevorzugt von 1 bis 6, weiter bevorzugt von 1 bis 4, ist und k, l jeweils unabhängig voneinander eine ganze Zahl von 0 bis 6, vorzugsweise von 1 bis 5, bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest ist, der a) nur ein neutrales, protonierbares Stickstoffatom enthält oder b) nur ein positiv geladenes Stickstoffatom, das vorzugsweise kein quartäres Stickstoffatom ist, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet,
   wobei der Reste R5 ein nichtcyclischer Polyolrest der allgemeinen Formel -CH₂(CH(OH))_{g}CH₂OH ist oder ein Ether, Ester oder Acetal davon, wobei g eine ganze Zahl von 1 bis 10, vorzugsweise 1 bis 4, bedeutet und
   wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 1 bis 20 C-Atomen, S-Alkenyl mit 1 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen,

Thioester mit 1 bis 20 C-Atomen, Alkyl mit 1 bis 20 C-Atomen, Alkenyl mit 1 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten.

Weiter bevorzugt ist der Reste R5 ein nichtcyclischer Polyolrest, der aus der Gruppe, die aus Arabityl, Ribityl, Xylityl, Erythrityl, Threityl, Lactityl, Mannityl und Sorbityl, weiter bevorzugt D-Ribityl und D-Arabityl, besteht, ausgewählt wird oder ein Ether, Ester oder Acetal davon.

Bei einer weiteren bevorzugten Ausführungsform des erfindungsgemäß als Photosensibilisator bei der Inaktivierung von Mikroorganismen verwendeten 10H-Benzo[g]pteridin-2,4-dion-Derivats der Formel (1) ist der Rest X ein Rest der Formel (5): 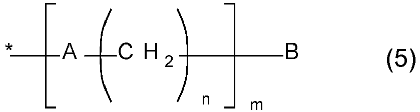 wobei A ein Sauerstoff- oder ein Schwefelatom ist und wobei n eine ganze Zahl von 1 bis 8 und m eine ganze Zahl von 0 bis 100, vorzugsweise von 0 bis 59, vorzugsweise von 0 bis 10, bedeutet und wobei B ein Rest der Formel (6a), (6b), (7) oder (8): 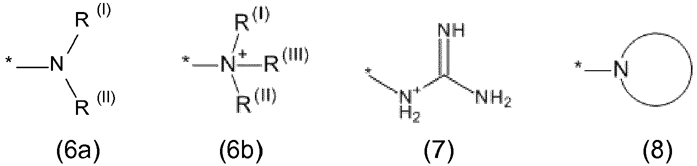 ist und wobei jeder der Reste R^{(I)} und R^{(II)} unabhängig voneinander ausgewählt wird aus Wasserstoff und C1 - C20 Alkyl, das geradkettig oder verzweigt sein kann, und wobei R^{(III)} Wasserstoff ist und wobei der Rest mit der Formel (8) 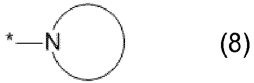 einen substituierten oder unsubstituierten heterocyclischen Rest mit 5 bis 7 Ringatomen, die mindestens ein 1 Kohlenstoffatom und 1 Stickstoffatom sowie optional 1 oder 2 Sauerstoff- oder Schwefelatom umfassen, wobei der heterocyclische Rest gesättigt oder ungesättigt ist und vorzugsweise keine quartären Stickstoffatome enthält, darstellt.

Bei einer weiteren bevorzugten Ausführungsform werden die Reste R^{(I)} und R^{(II)} unabhängig voneinander aus der Gruppe, die aus Wasserstoff und Alkylgruppen der allgemeinen Formel -(CH₂)ₙ-CH₃, wobei n eine ganze Zahl von 0 bis 19, vorzugsweise von 1 bis 17, ist, besteht, ausgewählt.

Bei einer weiteren bevorzugten Ausführungsform werden die Reste R^{(I)} und R^{(II)} unabhängig voneinander ausgewählt aus der Gruppe, die aus Wasserstoff, Methyl, Ethyl, Prop-1-yl, Prop-2-yl, But-1-yl, But-2-yl, 2-Methylprop-1-yl, 2-Methyl-prop-2-yl, Pent-1-yl, Pent-2-yl, Pent-3-yl, 2-Methylbut-1-yl, 2-Methylbut-2-yl, 2-Methylbut-3-yl, 2-Methylbut-4-yl, 2,2-Dimethylprop-1-yl, Hex-1-yl, Hex-2-yl, Hex-3-yl, Hept-1-yl, Oct-1-yl, 2-Methylpent-1-yl, 2-Methylpent-2-yl, 2-Methylpent-3-yl, 2-Methylpent-4-yl, 2-Methylpent-5-yl, 3-Methylpent-1-yl, 3-Methylpent-2-yl, 3-Methylpent-3-yl, 2,2-Dimethylbut-1-yl, 2,2-dimethylbut-3-yl, 2,2-Dimethylbut-4-yl, 2,3-Dimethylbut-1-yl und 2,3-Dimethylbut-2-yl besteht, ausgewählt. Bei einer besonders bevorzugten Ausführungsform werden die Reste R^{(I)} R^{(II)} und R^{(III)} unabhängig voneinander aus der Gruppe, die aus Methyl, Ethyl, Prop-1-yl, But-1-yl, Pent-1-yl, Hex-1-yl, Hept-1und Oct-1-yl besteht, ausgewählt.

Bei einer weiteren bevorzugten Ausführungsform sind die Reste R^{(I)} und R^{(II)} unabhängig voneinander Wasserstoff oder der Rest der Formel (11): 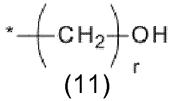 wobei r eine ganze Zahl von 1 bis 20, vorzugsweise von 1 bis 8, weiter bevorzugt von 1 bis 4, bedeutet.

Bei einer weiteren bevorzugten Ausführungsform wird der Rest der Formel (4): 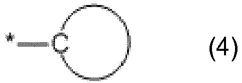 aus der Gruppe, die aus Resten der Formeln (13a), (13b) und (13c) 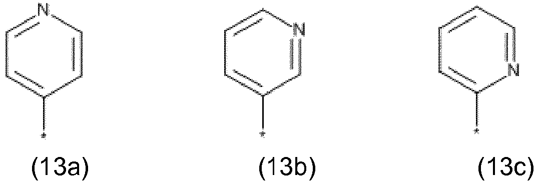 besteht, ausgewählt.

Bei einer weiteren bevorzugten Ausführungsform bedeutet der Rest mit der Formel (8): 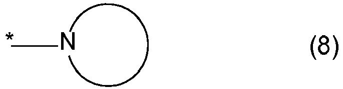 einen substituierten oder unsubstituierten heterocyclischen Rest mit 5 bis 7 Ringatomen, die mindestens ein 1 Kohlenstoffatom und 1 Stickstoffatom sowie optional 1 oder 2 Sauerstoff- oder Schwefelatom umfassen, wobei der heterocyclische Rest gesättigt oder ungesättigt ist und vorzugsweise keine quartären Stickstoffatome enthält.

Unter dem Begriff "heterocyclisch" werden erfindungsgemäß cyclische Verbindungen mit ringbildenden Atomen aus mindestens zwei verschiedenen chemischen Elementen, vorzugsweise Kohlenstoff, Stickstoff, Sauerstoff oder Schwefel, verstanden.

Bei einer weiteren bevorzugten Ausführungsform wird der Rest mit der Formel (4) ausgewählt aus der Gruppe, die aus Azolylen, Azolinylen, Azolidinylen, Thiazolylen, Oxazolylen, Oxazolinylen, Oxazolidinylen, Oxazinylen, Dihydrooxazinylen, Tetrahydrooxazinylen, Thiazinylen, Azepanylen, Azepinylen, oder Thiaazepinylen besteht, ausgewählt, wobei die vorgenannten heterocyclischen Reste unsubstituiert sind oder mit wenigstens einem Rest, der aus der Gruppe, die aus Halogen, vorzugsweise Chlor, Brom, Iod oder Fluor, Phenyl, Benzyl, geradkettigen oder verzweigten Alkyl mit 1 bis 20 C-Atomen und Hydroxyl besteht, ausgewählt wird, substituiert sein können und vorzugsweise keine quartären Stickstoffatome enthalten.

Bei einer weiter bevorzugten Ausführungsform wird der Rest mit der Formel (4) aus Verbindungen mit 5 bis 7 Ringatomen gebildet, wobei diese Verbindungen aus der Gruppe, die aus Pyrrolidin, Pyrrol, Oxazol, Oxazolin, Oxazolidin, Isoxazol, Isoxazolin, Isoxazolidin, Thiazol, Thiazolin, Thiazolidin, Isothiazol, Isothiazolin, Isothiazolidin, Piperidin, Pyridin, Oxazinen, Dihydrooxazinen, Tetrahydrooxazinen, vorzugsweise Morpholin, Thiazin, Azepinen, Azepan, und Thiazepinen besteht, ausgewählt wird, wobei die vorgenannten Verbindungen unsubstituiert sind oder mit wenigstens einem Rest, der aus der Gruppe, die aus Halogen, vorzugsweise Chlor, Brom, Iod oder Fluor, Phenyl, Benzyl, geradkettigen oder verzweigten Alkyl mit 1 bis 20 C-Atomen, und Hydroxyl besteht, ausgewählt wird, substituiert sein können, und vorzugsweise keine quartären Stickstoffatome enthalten.

Bei einer besonders bevorzugten Ausführungsform wird der Rest der Formel (4) ausgewählt aus der Gruppe, die aus Pyrrolidinyl, Piperidinyl, Morpholinyl, Pyridinyl, Aminopyridinyl, Azepan-1-yl, 2, 3, 4, 5-Tetrahydro-1-benzazepin-1-yl, 2, 3, 4, 5-Tetrahydro-3-benzazepin-3-yl.

Bei einer weiteren bevorzugten Ausführungsform bedeutet der Rest mit der Formel (8): 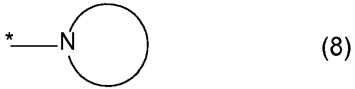 einen substituierten oder einen unsubstituierten cyclischen Heteroalkylrest mit 5 bis 7 Ringatomen, die mindestens ein 1 Kohlenstoffatom und 1 Stickstoffatom sowie optional 1 oder 2 Sauerstoff- oder Schwefelatom umfassen und vorzugsweise keine quartären Stickstoffatome enthalten, oder einen substituierten oder unsubstituierten cyclischen Heteroalkenylrest mit 5 bis 7 Ringatomen, die mindestens ein 1 Kohlenstoffatom und 1 Stickstoffatom sowie optional 1 oder 2 Sauerstoff- oder Schwefelatom umfassen, und vorzugsweise keine quartären Stickstoffatome enthalten.

Bei Variante A) und Variante B) des erfindungsgemäß als Photosensibilisator bei der Inaktivierung von Mikroorganismen verwendeten 10H-Benzo[g]pteridin-2,4-dion-Derivats der Formel (1) können vorgenannte Aldehydreste, Ketonreste, Carbonsäurereste, Carbonsäureamidreste, Thioesterreste, Cycloalkylreste, Cycloalkenylreste, Alkylreste und Akenylreste geradkettig oder verzweigt, vorzugsweise geradkettig, sein und sowohl unsubstituiert oder mit wenigstens einem Rest, der aus der Gruppe, die aus Hydroxy, Halogen, vorzugsweise Chlor, Brom, Iod oder Fluor, Thiol, Nitro, Ester, Ether und Acetal besteht, ausgewählt wird, substituiert sein.

Bei einer weiteren bevorzugten Ausführungsform werden die vorgenannte Alkylreste jeweils aus der Gruppe, die aus Methyl, Ethyl, n-Propyl, n-Butyl, n-Pentyl, n-Hexyl, n-Heptyl und n-Octyl besteht, ausgewählt.

Bei einer weiter bevorzugten Ausführungsform können vorgenannte Alkylreste nichtcyclische Polyolreste der allgemeinen Formel -CH₂(CH(OH))_{g}CH₂OH sein, wobei g eine ganze Zahl von 1 bis 10, vorzugsweise 1 bis 4, ist. Weiter bevorzugt werden die nichtcyclischen Polyolreste aus der Gruppe, die aus Arabityl, Ribityl, Xylityl, Erythrityl, Threityl, Lactityl, Mannityl und Sorbityl, weiter bevorzugt D-Ribityl und D-Arabityl, besteht, ausgewählt.

Bei einer bevorzugten Ausführungsform des erfindungsgemäß als Photosensibilisator bei der Inaktivierung von Mikroorganismen verwendeten 10H-Benzo[g]pteridin-2,4-dion-Derivats der Formel (1) weisen die Arylreste jeweils höchstens 4, weiter bevorzugt höchstens 3, weiter bevorzugt höchstens 2, anellierte Ringe auf. Noch weiter bevorzugt weisen die Arylreste jeweils 1 Ring auf.

Bei einer bevorzugten Ausführungsform des erfindungsgemäß als Photosensibilisator bei der Inaktivierung von Mikroorganismen verwendeten 10H-Benzo[g]pteridin-2,4-dion-Derivats der Formel (1) werden die vorgenannten Arylreste aus der Gruppe, die aus Phenyl, Benzyl, Naphtyl, Anthracenyl, Phenanthrenyl und Pyrenyl besteht, ausgewählt.

Bei einer bevorzugten Ausführungsform des erfindungsgemäß als Photosensibilisator bei der Inaktivierung von Mikroorganismen verwendeten 10H-Benzo[g]pteridin-2,4-dion-Derivats der Formel (1) weisen die vorgenannten Alkenylreste 2 bis 17 C-Atome, weiter bevorzugt 2 bis 13 C-Atome, weiter bevorzugt 2 bis 9 C-Atome, weiter bevorzugt 2 bis 5 C-Atome, auf. Bei einer weiter bevorzugten Ausführungsform werden die vorgenannten Alkenylreste aus der Gruppe, die aus Ethenyl, n-Propenyl und n-Butenyl besteht, ausgewählt.

Bei einer weiteren bevorzugten Ausführungsform des erfindungsgemäß als Photosensibilisator bei der Inaktivierung von Mikroorganismen verwendeten 10H-Benzo[g]pteridin-2,4-dion-Derivats der Formel (1) weisen die vorgenannten Aldehyde 1 bis 17 C-Atome, weiter bevorzugt 1 bis 13 C-Atome, weiter bevorzugt 1 bis 9 C-Atome, weiter bevorzugt 1 bis 5 C-Atome, auf. Bei einer weiter bevorzugten Ausführungsform werden die vorgenannten Aldehyde aus der Gruppe, die aus Methanal-1-yl (Formyl), Ethanal-1-yl (2-Oxoethyl), n-Propanal-1-yl (3-Oxopropyl) und n-Butanal-1-yl (4-Oxobutyl) besteht, ausgewählt.

Bei einer weiteren bevorzugten Ausführungsform des erfindungsgemäß als Photosensibilisator bei der Inaktivierung von Mikroorganismen verwendeten 10H-Benzo[g]pteridin-2,4-dion-Derivats der Formel (1) weisen die vorgenannten Ketone 2 bis 17 C-Atome, weiter bevorzugt 3 bis 14 C-Atome, weiter bevorzugt 3 bis 9 C-Atome, auf. Bei einer weiter bevorzugten Ausführungsform werden die vorgenannte Ketone aus der Gruppe, die aus Dimethylketyl, Methyl-Ethyl-ketyl, Ethyl-Methyl-ketyl, Diethylketyl, Methyl-Propyl-ketyl, Ethyl-Propyl-ketyl, Propyl-Methyl-ketyl, Propyl-Ethyl-ketyl und Dipropyl-ketyl, das geradkettig oder verzweigt sein kann, besteht, ausgewählt.

Bei einer weiter bevorzugten Ausführungsform können vorgenannte Aldehydreste und/oder Ketonreste Monosaccharidreste, vorzugsweise Pentose- oder Ketosereste, sein.

Vorzugsweise haben geeignete Monosaccharidreste 3 bis 7 Kohlenstoffatome, vorzugsweise 5 bis 6 Kohlenstoffatome, und weisen eine Carbonylgruppe, vorzugsweise Aldehydgruppe oder Ketogruppe, sowie mindestens eine Hydroxylgruppe auf und können offenkettig oder cyclisch, vorzugsweise als Furanose oder Pyranose, vorliegen.

Bevorzugt leiten sich geeignete Monosaccharidreste von Monosacchariden ab, die aus der Gruppe, die aus D-Glycerinaldehyd, L-Glycerinaldehyd, D-Erythrose, L-Erythrose, D-Threose, L-Threose, D-Ribose, L-Ribose, D-Arabinose, L-Arabinose, D-Xylose, L-Xylose, D-Lyxose, L-Lyxose, D-Allose, L-Allose, D-Altrose, L-Altrose, D-Glucose, L-Glucose, D-Mannose, L-Mannose, D-Gulose, L-Gulose, D-Idose, L-Idose, D-Galactose, L-Galactose, D-Talose, L-Talose, Dihydroxyaceton, D-Erythrulose, L-Erythrulose, D-Ribulose, L-Ribulose, D-Xylulose, L-Xylulose, D-Psicose, L-Psicose, D-Fructose, L-Fructose, D-Sorbose, L-Sorbose, D-Tagatose und L-Tagatose besteht, ausgewählt. Weiter bevorzugt werden geeignete Monosaccharide aus der Gruppe, die aus D-Ribose, L-Ribose, D-Arabinose, L-Arabinose, D-Xylose, L-Xylose, D-Lyxose, L-Lyxose, D-Allose, L-Allose, D-Altrose, L-Altrose, D-Glucose, L-Glucose, D-Mannose, L-Mannose, D-Gulose, L-Gulose, D-Idose, L-Idose, D-Galactose, L-Galactose, D-Talose, L-Talose, D-Ribulose, L-Ribulose, D-Xylulose, L-Xylulose, D-Psicose, L-Psicose, D-Fructose, L-Fructose, D-Sorbose, L-Sorbose, D-Tagatose und L-Tagatose besteht, ausgewählt werden.

Bei einer weiteren bevorzugten Ausführungsform des erfindungsgemäß als Photosensibilisator bei der Inaktivierung von Mikroorganismen verwendeten 10H-Benzo[g]pteridin-2,4-dion-Derivats der Formel (1) weisen die vorgenannten Carbonsäureester 1 bis 17 C-Atome, weiter bevorzugt 1 bis 15 C-Atome, weiter bevorzugt 1 bis 12 C-Atome, auf. Bei einer weiter bevorzugten Ausführungsform werden die vorgenannten Carbonsäureester aus der Gruppe, die aus Ethylester, n-Propylester, i-Propylester, n-Butylester, sec.-Butylester, tert.-Butylester und Benzylester besteht, ausgewählt.

Bei einer weiteren bevorzugten Ausführungsform des erfindungsgemäß als Photosensibilisator bei der Inaktivierung von Mikroorganismen verwendeten 10H-Benzo[g]pteridin-2,4-dion-Derivats der Formel (1) weisen die vorgenannten Carbonsäureamide 1 bis 17 C-Atome, weiter bevorzugt 1 bis 15 C-Atome, weiter bevorzugt 1 bis 12 C-Atome, auf. Bei einer weiter bevorzugten Ausführungsform werden die vorgenannten Carbonsäureamide aus der Gruppe, die aus Amid, N-Methylamid, N-Ethylamid, N-(n-Propyl)amid, N-(i-Propyl)amid, N-(n-Butyl)amid, N-(sec.-Butyl)amid, N-(tert.-Butyl)amid, N-Phenylamid, N-Benzylamid, N,N-Dimethylamid, N-Methyl-N-Ethyl-amid, N,N-Diethylamid, N-Methyl-N-(n-Propyl)amid, N-Methyl-N-(i-Propyl)amid, N-Methyl-N-(n-Butyl)amid, N-Methyl-N-(sec.-Butyl)amid, N-Methyl-N-(tert.-Butyl)amid, N-Ethyl-N-(n-Propyl)amid, N-Ethyl-N-(i-Propyl)amid, N-Ethyl-N-(n-Butyl)amid, N-Ethyl-N-(sec.-Butyl)amid, N-Ethyl-N-(tert.-Butyl)amid, N-(n-Propyl)-N-(n-Propyl)amid, N-(n-Propyl)-N-(i-Propyl)amid, N-(n-Propyl)-N-(n-Butyl)amid, N-(n-Propyl)-N-(sec.-Butyl)amid, N-(n-Propyl)-N-(tert.-Butyl)amid, N-(i-Propyl)-N-(n-Propyl)amid, N-(i-Propyl)-N-(i-Propyl)amid, N-(i-Propyl)-N-(n-Butyl)amid, N-(i-Propyl)-N-(sec.-Butyl)amid, N-(i-Propyl)-N-(tert.-Butyl)amid, N-(n-Butyl)-N-(n-Propyl)amid, N-(n-Butyl)-N-(i-Propyl)amid, N-(n-Butyl)-N-(n-Butyl)amid, N-(n-Butyl)-N-(sec.-Butyl)amid, N-(n-Butyl)-N-(tert.-Butyl)amid, N-(sec.-Butyl)-N-(n-Propyl)amid, N-(sec.-Butyl)-N-(i-Propyl)amid, N-(sec.-Butyl)-N-(n-Butyl)amid, N-(sec.-Butyl)-N-(sec.-Butyl)amid, N-(sec.-Butyl)-N-(tert.-Butyl)amid, N-(tert.-Butyl)-N-(n-Propyl)amid, N-(tert.-Butyl)-N-(i-Propyl)amid, N-(tert.-Butyl)-N-(n-Butyl)amid, N-(tert.-Butyl)-N-(sec.-Butyl)amid, N-(tert.-Butyl)-N-(tert.-Butyl)amid, N,N-Diphenylamid, N,N-Dibenzylamid, N-Phenyl-N-Benzylamid, N-Methyl-N-Phenylamid, N-Methyl-N-Benzylamid, N-Ethyl-N-Phenylamid, N-Ethyl-N-Benzylamid, N-Phenyl-N-(n-Propyl)amid, N-Phenyl-N-(i-Propyl)amid, N-Phenyl-N-(n-Butyl)amid, N-Phenyl-N-(sec.-Butyl)amid, N-Phenyl-N-(tert.-Butyl)amid, N-Benzyl-N-(n-Propyl)amid, N-Benzyl-N-(i-Propyl)amid, N-Benzyl-N-(n-Butyl)amid, N-Benzyl-N-(sec.-Butyl)amid und N-Benzyl-N-(tert.-Butyl)amid besteht, ausgewählt.

Bei einer weiteren bevorzugten Ausführungsform des erfindungsgemäß als Photosensibilisator bei der Inaktivierung von Mikroorganismen verwendeten 10H-Benzo[g]pteridin-2,4-dion-Derivats der Formel (1) wird der vorgenannten Heteroarylrest, der kein Stickstoffatom enthält, mit 4 bis 20 C-Atomen aus der Gruppe, die aus Thiophenyl, Furanyl, Benzothiofuranyl und Benzofuranyl besteht, ausgewählt.

Bei einer weiteren bevorzugten Ausführungsform des erfindungsgemäß als Photosensibilisator bei der Inaktivierung von Mikroorganismen verwendeten 10H-Benzo[g]pteridin-2,4-dion-Derivats der Formel (1) weisen die vorgenannten Etherreste 2 bis 17 C-Atome, weiter bevorzugt 2 bis 13 C-Atome, weiter bevorzugt 2 bis 9 C-Atome, auf. Bei einer weiter bevorzugten Ausführungsform werden die vorgenannten Etherreste beispielsweise aus der Gruppe, die aus Methoxymethyl, Methoxyethyl, Methoxy-n-propyl, Ethoxymethyl, n-Propoxymethyl, 2-Ethoxyethoxymethyl, 2-(2-Ethoxyethoxy)ethyl,, i-Propoxymethyl, tert.-Butyloxymethyl und Benzyloxymethyl besteht, ausgewählt..

Bei einer weiteren bevorzugten Ausführungsform des erfindungsgemäß als Photosensibilisator bei der Inaktivierung von Mikroorganismen verwendeten 10H-Benzo[g]pteridin-2,4-dion-Derivats der Formel (1) weisen die vorgenannten Thioetherreste 2 bis 17 C-Atome, weiter bevorzugt 2 bis 13 C-Atome, weiter bevorzugt 2 bis 9 C-Atome, auf. Bei einer weiter bevorzugten Ausführungsform werden die vorgenannten Thioetherreste beispielsweise aus der Gruppe, Methylsulfanylmethyl, Methylsulfanylethyl, 3-Methylsulfanyl-n-propyl, Ethylsulfanylmethyl, n-Propylsulfanylmethyl, 2-Ethylsulfanylethylsulfanylmethyl, 2-(2-ethylsulfanylethylsulfanyl)ethyl, 2-Methylsulfanylpropyl, tert.-Butylsulfanymethyl und Benzylsulfanymethyl besteht, ausgewählt.

Bei einer weiteren bevorzugten Ausführungsform weist das erfindungsgemäß als Photosensibilisator bei der Inaktivierung von Mikroorganismen verwendeten 10H-Benzo[g]pteridin-2,4-dion-Derivats der Formel (1) ein Molekulargewicht von weniger als 1300 g/mol, vorzugsweise weniger als 990 g/mol, weiter bevorzugt weniger als 810 g/mol, weiter bevorzugt weniger als 690 g/mol, noch weiter bevorzugt weniger als 610 g/mol, noch weiter bevorzugt weniger als 600 g/mol, noch weiter bevorzugt weniger als 570 g/mol, auf.

Chiralitätszentren können, wenn nicht anders angegeben, in der R- oder in der S-Konfiguration vorliegen. Die Erfindung betrifft sowohl die optisch reinen Verbindungen als auch Stereoisomerengemische, wie Enantiomerengemische und Diasteromerengemische, in jedem Verhältnis.

Die Erfindung betrifft vorzugsweise auch die Verwendung von Mesomeren und/oder Tautomeren der Verbindung mit der Formel (1), sowohl die Verwendung der reinen Verbindungen als auch die Verwendung von Isomerengemischen in jedem Verhältnis.

Bei einer weiteren bevorzugten Ausführungsform wird das erfindungsgemäß als Photosensibilisator bei der Inaktivierung von Mikroorganismen verwendeten 10H-Benzo[g]pteridin-2,4-dion-Derivats der Formel (1) aus der Gruppe, die aus den Verbindung mit den Formeln (14) bis (17) besteht, ausgewählt:

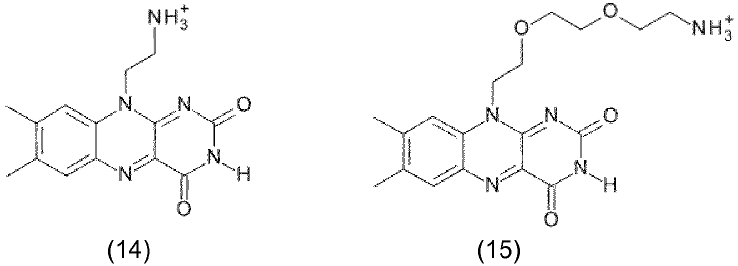

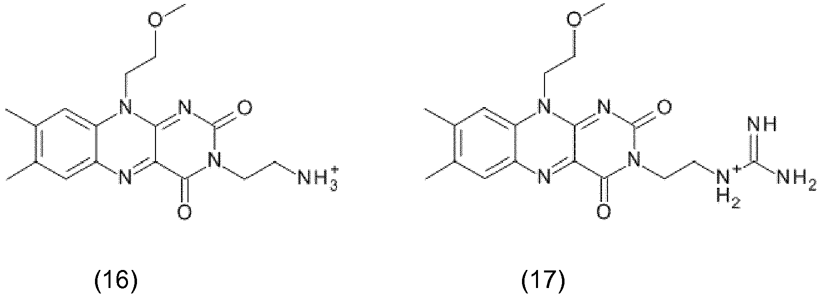

Die in der vorliegenden Anmeldung verwendeten 10H-Benzo[g]pteridin-2,4-dion-Derivate der Formel (1) können beispielsweise durch nachfolgend beschriebenes Verfahren hergestellt werden, wobei das Verfahren folgende Schritte umfasst:
(A) Reduzieren eines substituierten Nitroanilins der Formel (30) zu einem substituierten o-Phenylendiamin der Formel (31), vorzugsweise durch Wasserstoff und Palladium auf Aktivkohle oder mit Zinn(II)chlorid, 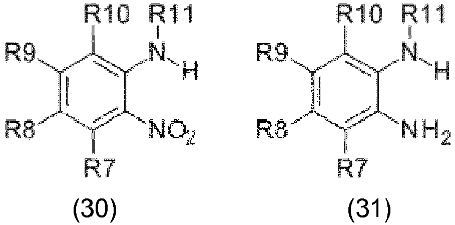 wobei jeder der Reste R7 bis R10, die jeweils unabhängig voneinander gleich oder voneinander verschieden sein können, Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH, - (C(D)(E))ₖ-Aryl-(C(D)(E))ₗ-OH, -(C(D)(E))ₕ-X oder -(C(D)(E))ₖ-Aryl-(C(D)(E))ₗ-X bedeutet und
   wobei der Rest R11 Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH oder -(C(D)(E))ₖ-Aryl-(C(D)(E))ₗ-OH, -(C(D)(E))ₕ-X oder -(C(D)(E))ₖ-Aryl-(C(D)(E))ₗ-X bedeutet,
(B) Kondensieren des in Schritt (A) erhaltenen substituierten o-Phenylendiamin der Formel (31) mit Alloxan oder dessen Hydrat unter Erhalt einer Verbindung mit der Formel (32), optional in Gegenwart eines Katalysators, vorzugsweise Lewis-Säure oder Broenstedt-Säure, weiter bevorzugt Essigsäure in Gegenwart von Borsäure, 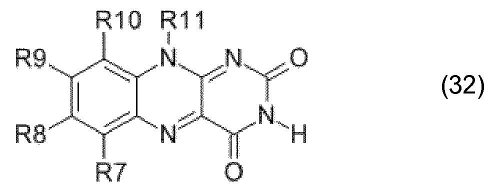
(C) Optional Umsetzen der in Schritt (B) erhaltenen Verbindung der Formel (32) mit einem Alkylierungsagens der allgemeinen Formel T-Alkyl, T-Alkenyl, T-Cycloalkyl, T-Cycloalkenyl, T-(C(D)(E))ₕ-OH, T-(C(D)(E))ₖ-Aryl-(C(D)(E))ₗ-OH, T-Aryl, T-(C(D)(E))ₕ-X oder T-(CH₂)ₖ-Aryl-(C(D)(E))ₗ-X, wobei der Rest T Wasserstoff, Chlor, Brom, Iod, p-Toluolsulfonyl (OTs), Methansulfonyl (OMs), OH oder R₂S⁺, wobei R jeweils unabhängig voneinander gleich oder verschieden sein kann und vorzugsweise Methyl, Ethyl, Propyl oder Butyl ist, bedeutet, unter Erhalt einer Verbindung mit der Formel (33): 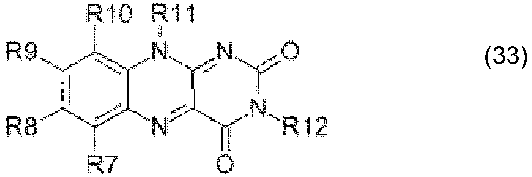
(D) Optional Umsetzen der in Schritt (B) erhaltenen Verbindung der Formel (32) oder der in Schritt (C) erhaltenen Verbindung der Formel (33) mit Tosylchlorid, Mesylchlorid oder Iod, optional in Gegenwart eines Katalysators, und nachfolgend mit einer organischen Verbindung, die wenigstens ein tertiäres Stickstoffatom enthält, wenn keiner der Reste R7 bis R12 ein organischer Rest der allgemeinen Formel - (C(D)(E))ₕ-X oder -(C(D)(E))ₖ-Aryl-(C(D)(E))ₗ-X ist und wenn 1 Rest R7 bis R12 ein organischer Rest der allgemeinen Formel -(C(D)(E))ₕ-OH oder -(C(D)(E))ₖ-Aryl-(C(D)(E))ₗ-OH ist, unter Erhalt des erfindungsgemäßen 10H-Benzo[g]pteridin-2,4-dion-Derivats der Formel (1),
   mit der Maßgabe, dass nur 1 Rest R7 bis R12 ein organischer Rest der allgemeinen Formel (2), (3) oder (4):

   -(C(D)(E))ₕ-X, (2)

   -(C(D)(E))ₖ-Aryl-(C(D)(E))ₗ-X, (3)

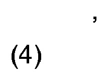

   ist,
   wobei h eine ganze Zahl von 1 bis 20 und k und l jeweils unabhängig voneinander eine ganze Zahl von 0 bis 6, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeuten und wobei X ein organischer Rest ist, der a) nur ein neutrales, protonierbares Stickstoffatom enthält oder b) nur ein positiv geladenes Stickstoffatom, das vorzugsweise kein quartäres Stickstoffatom ist, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   und wobei der Rest mit der Formel (4) ein Heteroaromat, der über ein C-Atom des Heteroaromaten an den Isoalloxazinring gebunden ist und der a) nur ein neutrales, protonierbares Stickstoffatom oder b) nur ein positiv geladenes Stickstoffatom enthält, bedeutet.

Die in der vorliegenden Anmeldung verwendeten 10H-Benzo[g]pteridin-2,4-dion-Derivate der Formel (1) können weiterhin durch nachfolgend beschriebenes Verfahren hergestellt werden, wobei das Verfahren folgende Schritte umfasst:
(A) Kondensieren eines Amins mit der Formel R11-NH₂ mit einem Chloruracil-Derivate der Formel (34), optional in Gegenwart eines Katalysators, vorzugsweise Lewis-Säure oder Broenstedt-Säure, unter Erhalt einer Verbindung mit der Formel (35): 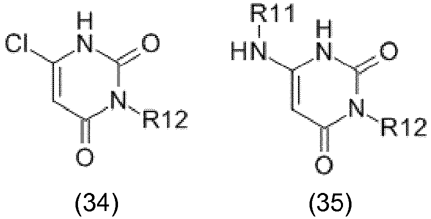 wobei jeder der Reste R11 oder R12 jeweils unabhängig voneinander gleich oder voneinander verschieden sein kann und Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH oder -(C(D)(E))ₖ-Aryl-(C(D)(E))ₗ-OH, -(C(D)(E))ₕ-X oder -(C(D)(E))ₖ-Aryl-(C(D)(E))ₗ-X bedeutet,
(B) Umsetzen der in Schritt (A) erhaltenen Verbindung der Formel (35) mit einer Nitrosoverbindung der Formel (36) unter Erhalt einer Verbindung der Formel (33): 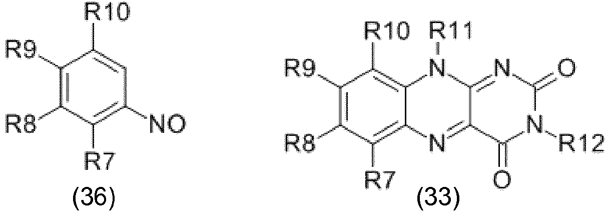 wobei jeder der Reste R7 bis R10 jeweils unabhängig voneinander gleich oder voneinander verschieden sein kann und Wasserstoff, Halogen, Hydroxyl, Thiol, Nitro, Carboxylat, Aldehyd mit 1 bis 20 C-Atomen, Keton mit 2 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 1 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen, Heteroaryl mit 4 bis 20 C-Atomen, das keine N-Atome enthält, oder ein organischen Rest der allgemeinen Formel -(C(D)(E))ₕ-OH, -(C(D)(E))ₖ-Aryl-(C(D)(E))ₗ-OH, -(C(D)(E))ₕ-X oder -(C(D)(E))ₖ-Aryl-(C(D)(E))ₗ-X bedeutet, und
(C) Optional Umsetzen der in Schritt (B) erhaltenen Verbindung der Formel (33) mit Tosylchlorid, Mesylchlorid oder Iod, optional in Gegenwart eines Katalysators, und nachfolgend mit einer organischen Verbindung, die wenigstens ein tertiäres Stickstoffatom enthält, wenn keiner der Reste R7 bis R12 ein organischer Rest der allgemeinen Formel -(C(D)(E))ₕ-X oder-(C(D)(E))ₖ-Aryl-(C(D)(E))ₗ-X ist und wenn 1 Rest R7 bis R12 ein organischer Rest der allgemeinen Formel -(C(D)(E))ₕ-OH oder -(C(D)(E))ₖ-Aryl-(C(D)(E))ₗ-OH ist, unter Erhalt des erfindungsgemäßen 10H-Benzo[g]pteridin-2,4-dion-Derivats der Formel (1),
   mit der Maßgabe, dass nur 1 Rest R7 bis R12 ein organischer Rest der allgemeinen Formel (2), (3) oder (4) ist,
   wobei h eine ganze Zahl von 1 bis 20 und k und l jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol, weiter bevorzugt Wasserstoff oder Hydroxyl, bedeutet und wobei X ein organischer Rest ist, der a) nur ein neutrales, protonierbares Stickstoffatom enthält oder b) nur ein positiv geladenes Stickstoffatom, das vorzugsweise kein quartäres Stickstoffatom ist, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
   und wobei der Rest mit der Formel (4) ein Heteroaromat, der über ein C-Atom des Heteroaromaten an den Isoalloxazinring gebunden ist und der a) nur ein neutrales, protonierbares Stickstoffatom oder b) nur ein positiv geladenes Stickstoffatom enthält, bedeutet.

Bei der Verwendung unterschiedlicher Amino-Schutzgruppe PG in einer Synthese, ergibt sich die Möglichkeit der orthogonalen Schutzgruppenstrategie, wobei unterschiedliche Amino-Funktionen eines Moleküls gezielt nacheinander freigesetzt können und zur Reaktion gebracht werden.

Geeignete Methoden zur Entfernung der Amino-Schutzgruppe PG sind aus dem Stand der Technik bekannt. Beispielsweise kann Benzyloxycarbonyl (Cbz) durch katalytische Hydrierung unter hydrogenolytischer Spaltung der Benzyl-Heteroatom-Bindung mit anschließender Decarboxylierung der so entstehenden instabilen Carbaminsäure oder Behandlung mit Säuren wieder entfernt werden. Di-tert-butyloxycarbonyl (Boc) kann beispielsweise durch saure Hydrolyse entfernt werden. Allyloxycarbonyl (Alloc) kann beispielsweise durch Einwirkung von Tetrakis(triphenylphosphan)palladium(0) und einem Nukleophil abgespalten werden.

Bei einer weiteren bevorzugten Ausführungsform finden die Schritte (B) und/oder (C) in Gegenwart eines oder mehrerer Lösungsmittel statt. Der Schritt (B) kann beispielsweise in Gegenwart von Dichlormethan (DCM), Dimethylformamid (DMF) oder Acetonitril (MeCN) durchgeführt werden. Der Schritt (C) kann beispielsweise in Gegenwart von Wasser/Dichlormethan oder Toluol/Tetrabutylammoiumiodid (TBAI) durchgeführt werden.

Einzellige oder mehrzellige Mikroorganismen können Auslöser von infektiösen Erkrankungen sein. Durch Applikation wenigstens eines erregerspezifischen Gegenmittels, beispielsweise Antibiotikum, Antimikotikum oder Virustatikum, kann die Anzahl der Erreger reduziert und/oder der Erreger inaktiviert werden. Die Applikation eines erregerspezifischen Gegenmittels kann systemisch und/oder topisch erfolgen.

Bei der systemischen Applikation wird das erregerspezifische Gegenmittel in das Blut- und/oder Lymphsystem des zu behandelnden Körpers übertragen und hierüber im gesamten Körper verteilt. Bei einer systemischen Aufnahme des erregerspezifischen Gegenmittels kann es zu einem Abbau des Gegenmittels und/oder zu Nebenwirkungen, beispielsweise durch eine biochemische Umwandlung (Metabolisierung) des Gegenmittels, kommen.

Bei der topischen Applikation des erregerspezifischen Gegenmittels erfolgt die Anwendung des Gegenmittels dort, wo es therapeutisch wirken soll, beispielsweise auf einer infizierten Hautpartie, während die gesunde Haut nicht belastet wird. Somit können systemische Nebenwirkungen weitgehend vermieden werden.

Oberflächliche Haut- oder Weichteilinfektionen müssen nicht notwendigerweise mit, einer systemischen Applikation eines erregerspezifischen Gegenmittels behandelt werden, da das Gegenmittel direkt auf die infizierte Hautpartien aufgetragen werden kann.

Die bisher bekannten erregerspezifischen Gegenmittel weisen sowohl bei systemischer als auch topischer Applikation teilweise starke Neben- und Wechselwirkungen auf. Darüber hinaus kann es auch bei topischer Applikation durch eine unzuverlässige Medikamenteneinnahme (Compliance) des Patienten, insbesondere bei Verwendung von Antibiotika, zur Resistenzbildung kommen.

Eine Alternative stellt hier die photodynamische Inaktivierung von Mikroorganismen dar, bei der Resistenzen gegenüber der photodynamischen Inaktivierung unbekannt sind. Unabhängig von der Art der zu bekämpfenden Mikroorganismen und der damit verbundenen infektiösen Erkrankungen wird die Anzahl der Erreger reduziert und/oder die Erreger werden abgetötet. Beispielsweise können Mischungen aus verschieden Mikroorganismen, beispielsweise Pilze und Bakterien oder unterschiedliche Bakterienstämme, bekämpft werden.

Bei einer bevorzugten Ausführungsform wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon als Photosensibilisator bei der photodynamischen Inaktivierung von Mikroorganismen, vorzugsweise bei der photodynamischen Therapie, verwendet.

Das erfindungsgemäß als Photosensibilisator bei der Inaktivierung von Mikroorganismen verwendete 10H-Benzo[g]pteridin-2,4-dion-Derivats der Formel (1) weist nach Bestrahlung mit elektromagnetischer Strahlung geeigneter Wellenlänge und Energiedichte eine hohe Ausbeute an Singulett-Sauerstoff auf.

Vorzugsweise liegt die elektromagnetische Strahlung im sichtbaren Spektralbereich, ultravioletten und/oder infraroten Bereich. Weiter bevorzugt weist die elektromagnetische Strahlung eine Wellenlänge aus einem Bereich von 280 bis 1000 nm, weiter bevorzugt von 380 bis 1000 nm, auf.

Weiter bevorzugt weist die elektromagnetische Strahlung eine Energiedichte aus einem Bereich von 1 µW/cm² bis 1 MW/cm², weiter bevorzugt von 1 mW/cm² bis 1 kW/cm², auf.

Die Bestrahlungszeit kann in Abhängigkeit von der Art der Mikroorganismen und/oder der Schwere der Infektion variiert werden. Vorzugsweise liegt die Bestrahlungszeit in einem Bereich von 1 µs bis 1 h, weiter bevorzugt von 1 ms bis 1000 s.

Vorzugsweise wird die elektromagnetische Strahlung durch eine Strahlungsquelle erzeugt, die aus der Gruppe, die aus Sonne und künstlichen Strahlungsquellen, beispielsweise UV-Lampe, IR-Lampe, Leuchtstofflampen, Leuchtdioden, Laser oder chemisches Licht, besteht, ausgewählt wird.

Darüber hinaus haben die Erfinder überraschenderweise festgestellt, dass ein erfindungsgemäß verwendetes 10H-Benzo[g]pteridin-2,4-dion-Derivat der Formel (1) oder ein pharmakologisch verträgliches Salz und/oder Ester und/oder Komplexe davon vorzugsweise eine hohe Affinität zu Mikroorganismen aufweist.

Auf Grund der Affinität kann das erfindungsgemäß verwendete 10H-Benzo[g]pteridin-2,4-dion-Derivats der Formel (1) an Mikroorganismen effektiv binden und lokal ausreichend Singulett-Sauerstoff erzeugen, um die Mikroorganismen zu inaktivieren, vorzugsweise abzutöten.

Bei dieser bevorzugten Verwendung als Photosensibilisator wird wenigstens ein 10H-Benzo[g]pteridin-2,4-dion-Derivat der Formel (1) von Mikroorganismen gebunden. Nach Bestrahlung mit elektromagnetischer Strahlung geeigneter Wellenlänge und Energiedichte werden die Mikroorganismen durch die entstandenen reaktiven Sauerstoffspezies (ROS), vorzugsweise Sauerstoffradikale und/oder Singulett-Sauerstoff, inaktiviert, vorzugsweise abgetötet.

Vorzugsweise erlaubt die Bindung wenigstens eines erfindungsgemäß verwendeten 10H-Benzo[g]pteridin-2,4-dion-Derivates der Formel (1) an Mikroorganismen ebenfalls eine Anfärbung oder Lokalisierung von Mikroorganismen. Dadurch kann vorzugsweise auch der Verlauf der Inaktivierung von Mikroorganismen oder der Dekolonisation verfolgt werden.

Erfindungsgemäß wird unter dem Begriff "Dekolonisation" das Entfernen, vorzugsweise vollständige Entfernen, von Mikroorganismen verstanden.

Bei einer weiteren bevorzugten Ausführungsform wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon bei der Inaktivierung von einzelligen oder mehrzelligen Mikroorganismen verwendet, die aus der Gruppe, die aus Viren, Archäeen, Bakterien, Bakteriensporen, Pilzen, beispielsweise Myzelpilze und Hefen, Pilzsporen, Protozoen, Algen und blutübertragbaren Parasiten besteht, ausgewählt werden.

Vorzugsweise können Körperoberflächen, beispielsweise Haut oder Schleimhaut, von Menschen und Tieren, vorzugsweise Säugetieren, behandelt werden. Bei dieser bevorzugten Ausführungsform wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon, vorzugsweise in einer pharmazeutischen Zubereitung, bei der Desinfektion und/oder Dekolonisierung von Haut- oder Weichteiloberflächen verwendet, wobei vorzugsweise die Hautintegrität erhalten bleibt.

Bei einer weiteren bevorzugten Ausführungsform ist wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon in einer pharmazeutischen Zubereitung zur topischen, vorzugsweise nasalen, oralen, analen, vaginalen oder dermalen, Applikation vorhanden.

Unter topischer Applikation wird auch die Anwendung am oder im Ohr, vorzugsweise Außenohr, verstanden. Das Außenohr umfasst den Ohrknorpel, die Ohrmuschel, das Ohrläppchen und den äußeren Gehörgang oder auch Ohrkanal und die Außenseite des Trommelfells.

Bei einer weiteren bevorzugten Ausführungsform wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon zur Herstellung einer pharmazeutischen Zubereitung bei der Prophylaxe und/oder Behandlung einer infektiösen, vorzugsweise viralen, bakteriellen und/oder mykotischen, Hautkrankheit, die vorzugsweise ausgewählt wird aus der Gruppe, die aus Staphylococcal scalded skin syndrome, Impetigo, Hautabszess, Furunkel, Karbunkel, Phlegmone, Cellulitis, Akute Lymphadenitis, Pilonidalzyste, Pyodermie, Dermatitis purulenta, Dermatitis septica, Dermatitis suppurativa, Erythrasma, Erysipelas, Akne vulgaris oder Pilzinfektion besteht, eingesetzt.

Bei einer weiteren bevorzugten Ausführungsform wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon zur Herstellung einer pharmazeutischen Zubereitung bei der Wundheilung, beispielsweise bei Heilungsstörungen nach chirurgischen Interventionen, eingesetzt.

Vorzugsweise wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon bzw. eine pharmazeutische Zubereitung enthaltend wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon bei der Desinfektion und/oder Reduktion der Keimzahl in infizierten Wunden eingesetzt.

Bei einer weiteren bevorzugten Ausführungsform wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon zur Herstellung einer pharmazeutischen Zubereitung bei der Prophylaxe und/oder Behandlung von infektiösen, vorzugsweise viralen, bakteriellen und/oder mykotischen, Erkrankungen des Ohrs, der oberen Luftwege, der Mundhöhle, des Rachens, des Kehlkopfes, der unteren Luftwege und/oder der Speiseröhre eingesetzt.

Das Vorherrschen pathogener Mikroorganismen ist beispielsweise die Hauptursache für Infektionen in der Mundhöhle. Dabei tritt das Problem auf, dass die Mikroorganismen in äußerst komplex aufgebauten Biofilmen synergetisch organisiert sind. Diese Biofilme, beispielsweise Plaque oder Zahnbelag, bestehen aus mehreren, komplex aufgebauten Schichten und enthalten Eiweiße, Kohlenhydrate, Phosphate und Mikroorganismen. Zahnbelag entsteht besonders dort, wo Zahnflächen nicht durch natürliche oder künstliche Reinigung belagfrei gehalten werden können. Dieser Umstand macht es schwierig, einen Zugang zu den im Biofilm eingebundenen Mikroorganismen zu finden.

Konventionelle Therapien, wie beispielsweise Antibiotika und Spüllösungen oder mechanische Zahnreinigung, können nur begrenzt eingesetzt werden, da sie entweder die Bakterien nicht direkt beeinflussen, beispielsweise bei der Zahnreinigung, nur schwer dosiert und appliziert werden können, beispielsweise bei Antibiotika und Spüllösungen, oder eine generelle Anwendung aufgrund von negativen Begleiterscheinungen nicht zu rechtfertigen ist.

Bei einer bevorzugten Ausführungsform wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon als Photosensibilisator bei der photodynamischen Inaktivierung von Mikroorganismen in der Mundhöhle eingesetzt.

Bei einer weiteren bevorzugten Ausführungsform wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon zur Herstellung einer pharmazeutischen Zubereitung bei der Behandlung und/oder Prophylaxe einer infektiösen, vorzugsweise viralen, bakteriellen und/oder mykotischen, Erkrankung des Zahngewebes, vorzugsweise Plaque, Karies oder Pulpitis, und/oder infektiösen, vorzugsweise viralen, bakteriellen und/oder mykotischen, Erkrankung des Zahnhalteapparates, vorzugsweise Gingivitis, Paradontitis, Endodontitis oder Periimplantitis, eingesetzt.

Bei einer weiteren bevorzugten Ausführungsform wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon bzw. eine pharmazeutische Zubereitung enthaltend wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon bei der Reinigung von Zähnen, Zahnersatz und/oder Zahnspangen eingesetzt.

Bei einer weiteren bevorzugten Ausführungsform wird wenigstens ein erfindungsgemäß verwendetes 10H-Benzo[g]pteridin-2,4-dion-Derivat oder ein pharmakologisch verträgliches Salz und/oder Ester und/oder Komplex davon bzw. eine pharmazeutische Zubereitung enthaltend wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon bei der nasalen Dekolonisierung von Mikroorganismen eingesetzt.

Beispielsweise besitzen Methicillin-resistente Staphylococcus aureus (MRSA)-Stämme eine monatelange Persistenz bei nasaler Kolonisierung sowie eine hohe Umweltresistenz. Daher reduziert eine nasale Dekolonisierung, d. h. Entfernung der Mikroorganismen, in der Regel auch die Kolonisation an anderen Körperstellen.

Des Weiteren betrifft die vorliegende Erfindung die Verwendung einer pharmazeutischen Zusammensetzung enthaltend wenigstens eines 10H-Benzo[g]pteridin-2,4-dion-Derivat der Formel (1) oder ein pharmakologisch verträgliches Salz und/oder Ester und/oder Komplex davon als Photosensibilisator bei der Inaktivierung von Mikroorganismen.

Vorzugsweise wird die pharmazeutische Zusammensetzung durch Mischen von mindestens einer Verbindung der Formel (1) oder ein pharmakologisch verträgliches Salz und/oder Ester und/oder Komplex davon mit einem oder mehreren physiologisch annehmbaren Hilfsstoff(en) hergestellt und in eine geeignete Darreichungsform gebracht.

Eine geeignete Darreichungsform der erfindungsgemäßen pharmazeutischen Zusammensetzung wird vorzugsweise aus der Gruppe, die aus Salbe, Creme, Gel, Lotion, Schüttelmixtur, Lösung, beispielsweise in Tropfen- oder Sprayform, Puder, Mikrokapsel und Paste besteht, ausgewählt.

Die erfindungsgemäße pharmazeutische Zusammensetzung kann topisch, vorzugsweise nasal, oral, anal, vaginal oder dermal, angewendet werden.

Als physiologisch annehmbare Hilfsstoffe kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage.

Bei einer weiteren bevorzugten Ausführungsform enthält die pharmazeutische Zusammensetzung eine effektive Menge wenigstens eines erfindungsgemäß verwendeten 10H-Benzo[g]pteridin-2,4-dion-Derivates der Formel (1) oder eines pharmakologisch verträglichen Salzes und/oder Esters und/oder Komplexes davon, wobei die effektive Menge von 0,01 µg bis 1000 µg pro Gramm der Zusammensetzung, vorzugsweise von 0,1 µg bis 500 µg pro Gramm der Zusammensetzung, umfasst.

Bei einer bevorzugten Ausführungsform der Erfindung umfasst die pharmazeutische Zusammensetzung wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon und wenigstens einen weiteren pharmazeutisch aktiven Bestandteil.

Vorzugsweise wird der wenigstens eine weitere pharmazeutisch aktive Bestandteil aus der Gruppe, die aus Antibiotika, Antimikotika, Virustatika, Antihistaminika, Sympathomimetika, Antihämorrhagika, Emmolientia und Hautschutzmittel, Analgetika, Desinfektionsmittel, Immunsera und Immunglobuline, Antiparasitäre Substanzen, Insektizide, Repellenzien und Corticosteroide besteht, ausgewählt.

Bei einer weiteren bevorzugten Ausführungsform wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon bzw. eine pharmazeutische Zubereitung enthaltend wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon durch den Anwender selbst aufgebracht und, optional, nachfolgend mit einer geeigneten Strahlenquelle, die elektromagnetische Strahlung geeigneter Wellenlänge und Energiedichte erzeugt, bestrahlt.

Bei einer weiteren bevorzugten Ausführungsform wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon bzw. eine Zubereitung enthaltend wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon bei der Inaktivierung von Mikroorganismen in medizinischen Blutprodukten verwendet.

Bei einer bevorzugten Ausführungsform wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon zur Inaktivierung von Mikroorganismen auf Oberflächen aller Art verwendet. Weiter bevorzugt wird das erfindungsgemäß verwendete 10H-Benzo[g]pteridin-2,4-dion-Derivat bei der Oberflächenreinigung und/oder -beschichtung, vorzugsweise von Medizinprodukten, Lebensmittelverpackungen oder Hygieneartikeln, verwendet.

Weiter bevorzugt wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon auf Oberflächen aufgebracht und/oder eingebracht und, optional, nachfolgend mit einer geeigneten Strahlenquelle, die elektromagnetische Strahlung geeigneter Wellenlänge und Energiedichte erzeugt, bestrahlt. Vorzugsweise bewirkt das wenigstens eine erfindungsgemäß verwendete 10H-Benzo[g]pteridin-2,4-dion-Derivat der Formel (1) oder ein pharmakologisch verträgliches Salz und/oder Ester und/oder Komplex davon während der Bestrahlung eine "Selbstdesinfektion" der Oberfläche.

Die Bestrahlung kann dabei direkt nach der Behandlung der Oberfläche mit wenigstens einem erfindungsgemäß verwendeten 10H-Benzo[g]pteridin-2,4-dion-Derivat der Formel (1) oder ein pharmakologisch verträgliches Salz und/oder Ester und/oder Komplex davon, vorzugsweise nach dem Aufbringen des wenigstens einen erfindungsgemäß verwendeten 10H-Benzo[g]pteridin-2,4-dion-Derivates der Formel (1) oder ein pharmakologisch verträgliches Salz und/oder Ester und/oder Komplex davon auf die Oberfläche und/oder Einbringen des wenigstens einen erfindungsgemäßen 10H-Benzo[g]pteridin-2,4-dion-Derivates der Formel (1) oder ein pharmakologisch verträgliches Salz und/oder Ester und/oder Komplex davon in die Oberfläche, erfolgen und/oder zu einem späteren Zeitpunkt.

Weiter bevorzugt werden Gegenstände behandelt, die eine thermisch begrenzte Haltbarkeit aufweisen, beispielsweise Gegenstände aus thermoplastischen Kunststoffen, oder von Desinfektionsmitteln angegriffen werden.

Gegenstände, die eine thermisch begrenzte Haltbarkeit aufweisen, können beispielsweise nur unzureichend sterilisiert werden, da sie bei höheren Temperaturen ihre Form verlieren oder spröde werden.

Darüber hinaus kann es bei einer unsachgemäßen und/oder übermäßigen Anwendung von Desinfektionsmitteln zu Resistenzbildung durch Selektion robuster Mikroorganismen kommen, wenn beispielsweise die Wirkstoffkonzentration und Einwirkzeit und damit die keimreduzierende Wirkung zu gering ist.

Bei einer weiter bevorzugten Ausführungsform wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon zur Inaktivierung von Mikroorganismen auf Oberflächen von Medizinprodukten, vorzugsweise invasiven medizinischen Hilfsmitteln wie etwa Kathetern, Hohlsonden, Schläuchen oder Nadeln, verwendet.

Vorzugsweise werden die Medizinprodukte aus Wundauflagen, Verbänden, Kathetern, Hohlsonden, Schläuchen oder Nadeln ausgewählt.

Weiter bevorzugt werden unter Medizinprodukten auch zahnärztliche Abdrücke oder Zahnersatz, beispielsweise Prothesen, Kronen oder Implantate, verstanden.

Vorzugsweise wird durch eine Behandlung der Oberfläche von Medizinprodukten mit wenigstens einem erfindungsgemäß verwendeten 10H-Benzo[g]pteridin-2,4-dion-Derivat der Formel (1) oder ein pharmakologisch verträgliches Salz und/oder Ester und/oder Komplex davon und/oder Beschichtung und/oder Immobilisierung wenigstens eines erfindungsgemäß verwendeten 10H-Benzo[g]pteridin-2,4-dion-Derivates auf der Oberfläche von Medizinprodukten und nachfolgender Bestrahlung mit elektromagnetischer Strahlung geeigneter Wellenlänge und Energiedichte die Besiedelung von Mikroorganismen auf der behandelten Oberflächen reduziert, vorzugsweise verhindert.

Die Bestrahlung kann dabei direkt nach der Behandlung der Oberfläche mit wenigstens einem erfindungsgemäß verwendeten 10H-Benzo[g]pteridin-2,4-dion-Derivat der Formel (1) oder ein pharmakologisch verträgliches Salz und/oder Ester und/oder Komplex davon, vorzugsweise nach dem Aufbringen des wenigstens einen erfindungsgemäß verwendeten 10H-Benzo[g]pteridin-2,4-dion-Derivates der Formel (1) oder ein pharmakologisch verträgliches Salz und/oder Ester und/oder Komplex davon auf die Oberfläche und/oder Einbringen des wenigstens einen erfindungsgemäß verwendeten 10H-Benzo[g]pteridin-2,4-dion-Derivates der Formel (1) oder ein pharmakologisch verträgliches Salz und/oder Ester und/oder Komplex davon in die Oberfläche, erfolgen und/oder zu einem späteren Zeitpunkt, vor oder während der Verwendung des behandelten Medizinproduktes.

Bei einer weiter bevorzugten Verwendung des wenigstens einen erfindungsgemäß verwendeten 10H-Benzo[g]pteridin-2,4-dion-Derivates der Formel (1) oder ein pharmakologisch verträgliches Salz und/oder Ester und/oder Komplex davon oder eines pharmakologisch verträgliches Salzes und/oder Esters und/oder Komplexes davon in Wundauflagen und/oder Verbänden, beispielsweise Baumwollgaze, kann während oder nach dem Aufbringen einer Wundauflage und/oder Verbandes, die bzw. der wenigstens ein erfindungsgemäß verwendetes 10H-Benzo[g]pteridin-2,4-dion-Derivat oder ein pharmakologisch verträgliches Salz und/oder Ester und/oder Komplex davon enthält, eine Bestrahlung mit elektromagnetischer Strahlung geeigneter Wellenlänge und Energiedichte erfolgen, wodurch es nachfolgend zu einer Reduzierung, vorzugsweise Inaktivierung, von Mikroorganismen im Wundbereich oder behandelten Hautpartien kommen.

Bei einer weiteren bevorzugten Ausführungsform umfasst die Wundauflage und/oder Verband neben wenigstens einem erfindungsgemäß verwendeten 10H-Benzo[g]pteridin-2,4-dion-Derivat der Formel (1) oder ein pharmakologisch verträgliches Salz und/oder Ester und/oder Komplex davon oder eines pharmakologisch verträgliches Salzes und/oder Esters und/oder Komplexes davon weitere Bestandteile, vorzugsweise Absorbtionsmittel, beispielsweise Calciumalginat oder Polyurethanschaum, oder weitere pharmazeutisch wirksame Substanzen.

Bei einer weiteren bevorzugten Ausführungsform wird wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon zur Inaktivierung von Mikroorganismen auf Oberflächen von Lebensmittelverpackungen verwendet.

Bei einer weiteren bevorzugten Ausführungsform wird wenigstens ein erfindungsgemäß verwendetes 10H-Benzo[g]pteridin-2,4-dion-Derivat zur Inaktivierung von Mikroorganismen in einer Flüssigkeit oder flüssigen, vorzugsweise wässrigen, Zubereitung, beispielsweise Dispersionsfarbe, verwendet.

Vorzugsweise handelt es sich bei der Flüssigkeit um Wasser.

Dabei kann wenigstens ein erfindungsgemäß verwendetes 10H-Benzo[g]pteridin-2,4-dion-Derivat zur Aufbereitung von Wasser für die Getränke- und Lebensmittelindustrie, die Pharma-, Chemie- und Kosmetikindustrie, die Elektroindustrie verwendet werden. Ferner kann wenigstens ein erfindungsgemäßes 10H-Benzo[g]pteridin-2,4-dion-Derivat der Formel (1) oder ein pharmakologisch verträgliches Salz und/oder Ester und/oder Komplex davon bei der Trinkwasser- und Regenwasseraufbereitung, der Behandlung von Abwasser oder bei der Aufbereitung von Wasser für den Einsatz in der Klimatechnik eingesetzt werden.

Bei dieser bevorzugten Verwendung wenigstens eines erfindungsgemäß verwendeten 10H-Benzo[g]pteridin-2,4-dion-Derivates der Formel (1) oder ein pharmakologisch verträgliches Salz und/oder Ester und/oder Komplex davon kann die Flüssigkeit oder flüssige Zubereitung nachfolgend mit einer geeigneten Strahlenquelle, die elektromagnetische Strahlung geeigneter Wellenlänge und Energiedichte erzeugt, bestrahlt werden. Vorzugsweise bewirkt das erfindungsgemäß verwendete 10H-Benzo[g]pteridin-2,4-dion-Derivat der Formel (1) oder ein pharmakologisch verträgliches Salz und/oder Ester und/oder Komplex davon während der Bestrahlung eine "Selbst-desinfektion" der Flüssigkeit oder der flüssigen Zubereitung.

Bei einer weiteren bevorzugten Verwendung des wenigstens einen erfindungsgemäß verwendeten 10H-Benzo[g]pteridin-2,4-dion-Derivates der Formel (1) oder ein pharmakologisch verträgliches Salz und/oder Ester und/oder Komplex davon kann das 10H-Benzo[g]pteridin-2,4-dion-Derivat an einen festen Träger gebunden vorliegen und so als Teil einer festen Matrix verwendet werden. Besonders bevorzugt wird wenigstens ein an einen festen Träger gebundenes erfindungsgemäß verwendetes 10H-Benzo[g]pteridin-2,4-dion-Derivat der Formel (1) oder ein pharmakologisch verträgliches Salz und/oder Ester und/oder Komplex davon in die zu behandelnde Flüssigkeit, vorzugsweise Wasser oder Blut, eingebracht.

Besonders bevorzugt ist als Träger ein Polymer, welches wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon daran in kovalent gebundener Weise trägt. Diese Zusammensetzung, umfassend den Träger und wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon, entwickelt eine antimikrobielle Aktivität, sobald sie elektromagnetischer Strahlung geeigneter Wellenlänge und Energiedichte ausgesetzt wird.

Des Weiteren betrifft die vorliegende Erfindung einen beschichteten Gegenstand, der wenigstens ein erfindungsgemäß verwendetes 10H-Benzo[g]pteridin-2,4-dion-Derivat enthält und/oder damit beschichtet ist.

Vorzugsweise weist die Oberfläche des beschichteten Gegenstands wenigstens ein erfindungsgemäßes 10H-Benzo[g]pteridin-2,4-dion-Derivat der Formel (1) oder ein pharmakologisch verträgliches Salz und/oder Ester und/oder Komplex davon auf.

Der beschichtete Gegenstand kann nachfolgend mit einer geeigneten Strahlenquelle, die elektromagnetische Strahlung geeigneter Wellenlänge und Energiedichte erzeugt, bestrahlt werden. Vorzugsweise bewirkt das erfindungsgemäß verwendete 10H-Benzo[g]pteridin-2,4-dion-Derivat der Formel (1) oder ein pharmakologisch verträgliches Salz und/oder Ester und/oder Komplex davon während der Bestrahlung eine "Selbst-desinfektion" der Oberfläche des beschichteten Gegenstandes.

Die Bestrahlung kann dabei direkt nach der Behandlung des beschichteten Gegenstandes mit wenigstens einem erfindungsgemäß verwendeten 10H-Benzo[g]pteridin-2,4-dion-Derivats der Formel (1) oder einem pharmakologisch verträglichen Salz und/oder Ester und/oder Komplex davon, vorzugsweise nach dem Aufbringen des wenigstens einen erfindungsgemäß verwendeten 10H-Benzo[g]pteridin-2,4-dion-Derivates der Formel (1) oder ein pharmakologisch verträgliches Salz und/oder Ester und/oder Komplex davon auf die Oberfläche des beschichteten Gegenstandes und/oder Einbringen des wenigstens einen erfindungsgemäß verwendeten 10H-Benzo[g]pteridin-2,4-dion-Derivates der Formel (1) oder ein pharmakologisch verträgliches Salz und/oder Ester und/oder Komplex davon in die Oberfläche des beschichteten Gegenstandes, erfolgen und/oder zu einem späteren Zeitpunkt, vorzugsweise vor oder während der Verwendung des beschichteten Gegenstandes.

Geeignete Gegenstände werden vorzugsweise aus der Gruppe, die aus Medizinprodukten, Lebensmittelverpackungen oder Hygieneartikeln, besteht, ausgewählt.

Bei einer weiteren bevorzugten Ausführungsform des beschichteten Gegenstandes handelt es sich um mit wenigstens einem erfindungsgemäß verwendeten 10-H-Benzo[g]pteridin-2,4-dion-Derivat der Formel (1) oder ein pharmakologisch verträgliches Salz und/oder Ester und/oder Komplex davon beschichtete Partikel, beispielsweise anorganische oder organische Partikel.

Weiter bevorzugt umfassen die Partikel wenigstens ein erfindungsgemäß verwendetes 10H-Benzo[g]pteridin-2,4-dion-Derivat der Formel (1) oder ein pharmakologisch verträgliches Salz und/oder Ester und/oder Komplex davon, das an die Partikel kovalent gebunden vorliegt.

Die Erfindung wird nachfolgend durch Figuren und Beispiele erläutert, ohne hierauf beschränkt zu sein.

### Synthese der verwendeten Verbindungen:

Alle Substanzen wurden analog literaturbekannter Vorschriften hergestellt:
1,2-Dinitro-4,5-dimethyl-benzen (31) gemäß: A. Monge, J. A. Palop, A. Lopez de Cerain, V. Senador, F.J. Martinez-Crespo, Y. Sainz, S. Narro, E. Garcia, C. de Miguel, M. Gonzälez, E. Hamilton, A.J. Barker, E.D. Clarke, D.T. Greenhow, J. Med. Chem. 1995, 38, 1786-1792; T. Sugaya, K. Nobuyuki, A. Sakaguchi, S. Tomioka, Synthesis 1995, 1257-1262; R.R. Holmes, R.P. Bayer, J. Am. Chem. Soc. 1960, 82, 3454-3456,
N-[2-({[(tert-Butyl)oxy]carbonyl}amino)ethyl]-4,5-dimethyl-2-nitro-anilin (32) gemäß J. Butenandt, R. Epple, E.-U. Wallenborn, A.P.M. Eker, V. Gramlich, T. Carell, Chem. Eur. J. 2000, 6, No. 1, 62-72,
N-(3'-Oxabut-1'-yl)-4,5-dimethyl-2-nitroanilin (**33**) und 10-(2'-Methoxyethyl)-7,8-dimethyl-10H-benzo[g]pteridine-2,4-dion (**37**) gemäß R. Epple, E.-U. Wallenborn, T. Carell, J. Am. Chem. Soc. 1997, 119, 7440-7451,
10-Aminoethyl-7,8-dimethyl-10H-benzo[g]pteridin-2,4-dion hydrochlorid (**39**) gemäß J. Butenandt, R. Epple, E.-U. Wallenborn, A.P.M. Eker, V. Gramlich, T. Carell, Chem. Eur. J. 2000, 6, No. 1, 62-72,
sowie 10-(8'-Amino-3',6'-dioxaoct-1'-yl)-7,8-dimethylbenzo[g]pter-idin-2,4-dion hydrochlorid (**40**) und 3-(2'-Aminoeth-1'-yl)-7,8-dimethyl-10-(3"-oxabut-1"-yl)benzo-[g]pteridin-2,4-dion (**42**) gemäß J. Svoboda, H. Schmaderer, B. König, Chem. Eur. J. 2008, 14, 1854 - 1865.

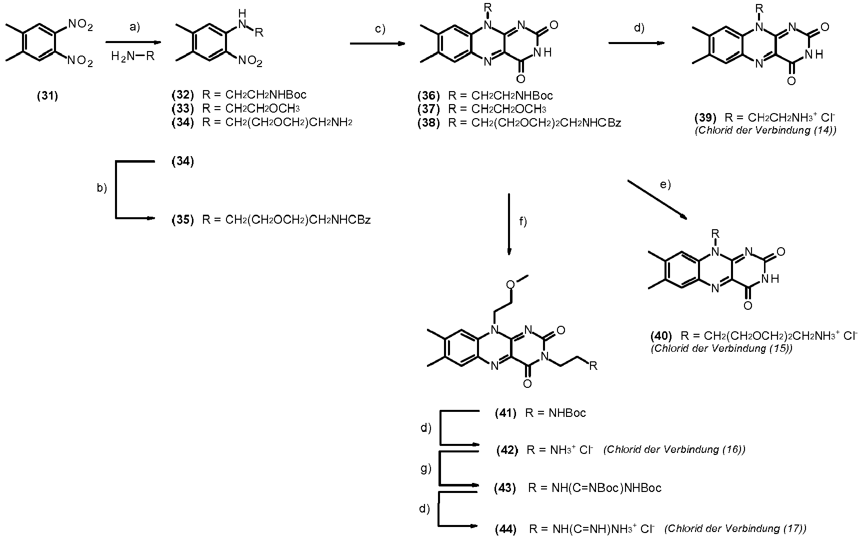

Bei der Synthese der Verbindungen (39), (40), (42) und (44) wurden folgende Reaktionsbedingungen gewählt:
a) EtOH, NEt₃, reflux, 2d; b) Cbz-Cl, DCM, NEt₃, 30 min., 0°C; c) Pd/C, H₂, HOAc, 14h, dann Alloxan monohydrat, H₃BO₃, HOAc, RT, 2d; d) HCl, Et₂O, DCM, RT, 10 h; e) ZnBr₂, HOAc, RT, über Nacht; HCl, Gefriertrocknung; f) 2-N-Boc-Aminoethylbromid, Cs₂CO₃, DMF, RT, 2d; g) 1,3-N,N'-di-Boc-2-methylisothioharnstoff, DCC, NEt₃, DMF, 2d, RT;

### Phototoxizitätsexperimente

### a) Herstellung der Testplatten und Bakterienstämme

Eine Probe des Bakterienstammes *Staphylococcus. aureus* (ATCC-Nummer: 25923) *oder Escherichia. coli* (ATCC-Nummer: 25922) wurde aus einer Cryo-Gefrierkultur entnommen, auf Müller-Hinton-Agar-Platten vereinzelt und unter aeroben Bedingungen bei 37°C in einer Übernachtkultur kultiviert. Daran anschließend wurden 5 ml Müller-Hinton-Flüssigmedium mit einem Abstrich der Bakterienkultur (Einzelkolonie) beimpft und über Nacht bei 37° C bebrütet. Die so gewonnene Bakteriensuspension wurde 10 min bei 2500 Upm zentrifugiert und das erhaltene Bakterienpellet in 5 ml sterilem PBS resuspendiert. Die optische Dichte der Bakteriensupensionen für die Phototoxizitätstests betrug OD₆₀₀ₙₘ= 0.6, was einer Bakterienzahl von ~1-8×10⁸⁻¹² Bakterien pro ml entspricht. Die biochemische Analyse und Resistenzbestimmung der Bakterien wurde mit dem VITEK2-System gemäß den Richtlinien M100-S14 des NCCLS (2004) durchgeführt.

Zur Empfindlichkeitsprüfung medizinisch bedeutender Krankheitserreger gegenüber Antibiotika und Sulfonamiden wurden gemäß der NCCLS Richtlinien Müller-Hinton-Medien verwendet (Deutsche Gesellschaft für Hygiene und Mikrobiologie (DGHM), Institut für Hygiene und Mikrobiologie, Universität Bonn, Deutschland):
a) Müller-Hinton-Bouillon (Oxoid, Wesel, Deutschland)
   2,0 g/l Rindfleisch, getrocknete Infusion aus 300 g, 17,5 g/l Caseinhydrolysat,
   1,5 g/l Stärke, pH: 7,4 + 0,2
b) Müller Hinton-Agar (Oxoid, Wesel, Deutschland)
   2,0 g/l Rindfleisch, getrocknete Infusion aus 300 g, 17,5 g/l Caseinhydrolysat,
   1,5 g/l Stärke, pH: 7,4 + 0,2
   13 g/l Agaragar

### b) Durchführung des Phototoxizitätstests:

200 µl einer Bakteriensuspension (Bakteriendichte: 10⁸- 10¹²/ml) wurden mit je 200 µl verschiedener Konzentrationen der zu testenden Photosensibilisatoren bei Raumtemperatur für 10 min oder 30 min inkubiert. Danach wurden die Bakterien zweimal mit Aqua dest. gewaschen, in 200 µl Aqua dest. resuspendiert, das gesamte Volumen auf eine 96-well Mikrotiterplatte übertragen und anschließend bestrahlt. Die verwendeten Photosensibilisatoren wurden in Aqua dest. gelöst und verschiedene Verdünnungsreihen hergestellt (0µM, 1µM, 10µM, 100µM).

Zur Sensibilisierung wurde die Lampe Omnicure Series 2000 (Photonics Solutions Inc., Edinburgh, UK) benutzt, die Licht aus einem Bereich von 390 nm bis 500nm emittiert und Emissionsmaxima Eₘₐₓ bei 405 nm und 436 nm aufweist. Die applizierte Leistung betrug jeweils 50mW/cm².

Als Kontrollen wurden bestrahlte und nicht bestrahlte Proben verwendet. Ebenso wurden nur mit Photosensibilisator inkubierte Bakteriensuspensionen mitgeführt (Dunkelkontrolle).

Die Bestimmung der koloniebildenden Einheiten (KBE bzw. CFU) pro ml wurde gemäß der von Miles und Misra publizierten Methode durchgeführt (Miles, AA; Misra, SS, Irwin, JO (1938 Nov). "The estimation of the bactericidal power of the blood.". The Journal of hygiene 38 (6): 732-49). Dazu wurden serielle Verdünnungen von 10⁻² bis 10⁻⁹ der entsprechenden Bakteriensuspension hergestellt. Je 3x 20 µl der entsprechenden Bakterienverdünnungen wurden dann auf Müller-Hinton Platten aufgetropft und bei 37° C für 24 h inkubiert. Danach wurde die Anzahl der überlebenden koloniebildenden Einheiten (KBE bzw. CFU) bestimmt. Alle Versuche wurden jeweils dreimal wiederholt.

### C) Ergebnis der Phototoxizitätsexperimente:

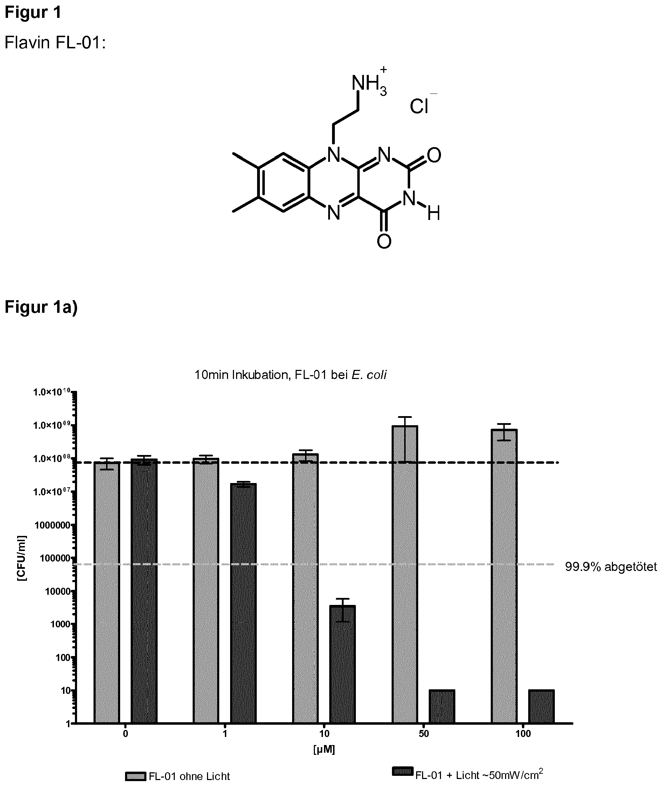

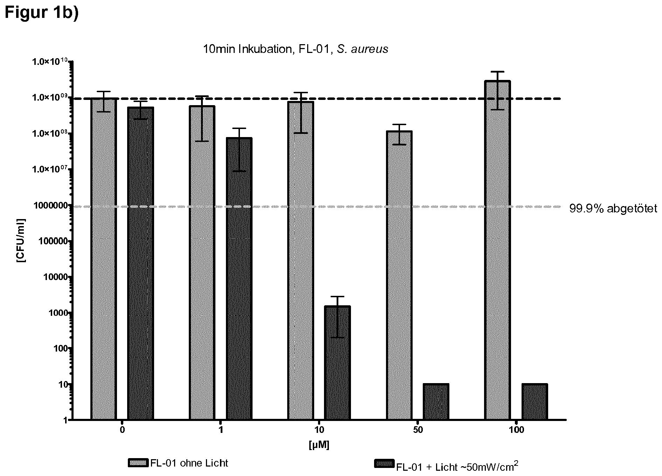

Die Ergebnisse der Phototoxizitätsexperimente sind in den Figuren 1-4 dargestellt.

Die Figuren 1-4 zeigen die logarithmische Abnahmen der CFU/ml 24 Std nach Bestrahlung sowie die dazugehörigen Kontrollen (nur bestrahlte Bakterien; mit Photosensibilisator inkubierte Bakterien, aber nicht bestrahlt; unbehandelte Bakterien) für den jeweils angegeben Photosensibilisator.

Die angegebenen koloniebildenden Einheiten (KBE bzw. CFU) pro ml sind jeweils der Median aus drei Versuchen.

Figur 1 zeigt die Wirkung von Flavin FL-01 (Chlorid der Verbindung mit der Formel (14)) auf E. coli und S. aureus.

Figur 1a: E. coli Proben wurden mit verschiedenen Konzentrationen (0 µM, 1 µM, 10 µM, 50 µM, 100 µM) von Flavin FL-01 für 10 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-01, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

Fig 1b S. aureus Proben wurden mit verschiedenen Konzentrationen (0 µM, 1 µM, 10 µM, 50 µM, 100 µM) von Flavin FL-01 für 10 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-01, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

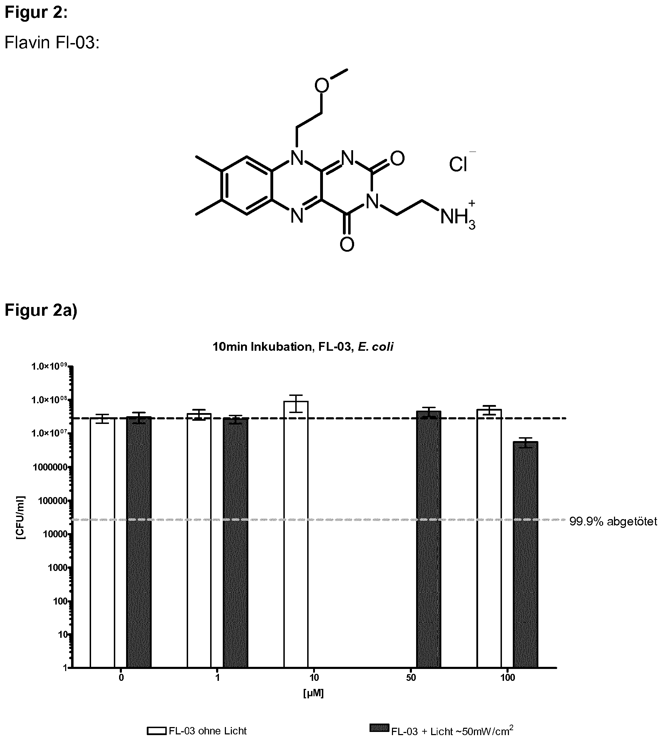

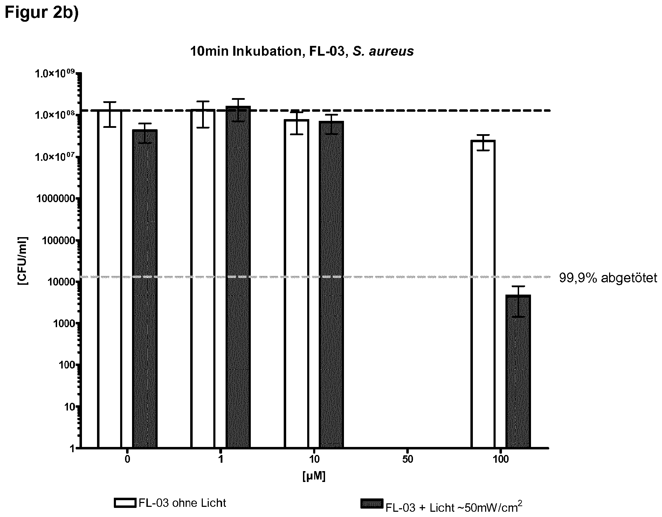

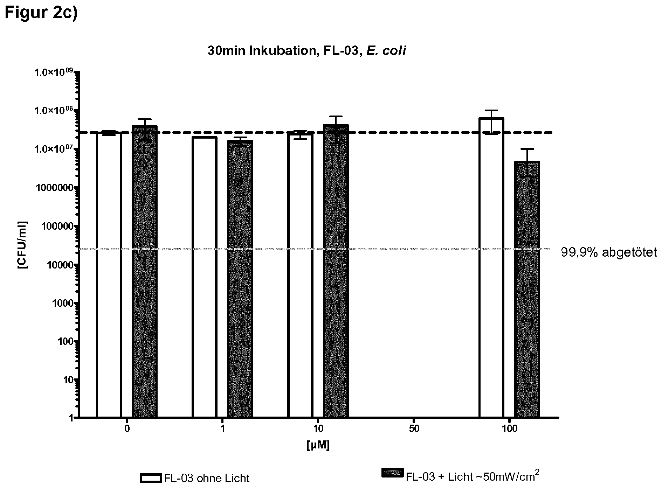

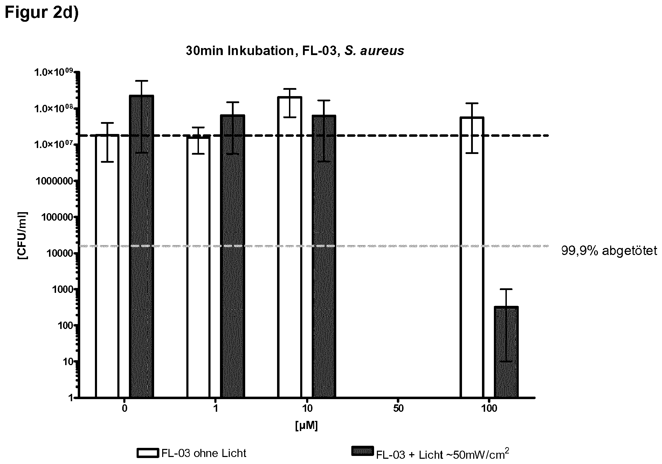

Figur 2 zeigt die Wirkung von Flavin FL-03 (Chlorid der Verbindung mit der Formel (16)) auf E. coli und S. aureus.

Figur 2a: E. coli Proben wurden mit verschiedenen Konzentrationen (0 µM, 1 µM, 10 µM, 50 µM, 100 µM) von Flavin FL-03 für 10 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-03, kein Licht). Die gestrichelte Linie, die mit der Bezeichnung "99.9% abgetötet" versehen ist, kennzeichnet eine Abnahme der CFU/ml um 3-log₁₀-Stufen; dies entspricht einer Abnahme um 99.9% ("antibakterielle Wirkung"). N= 3; Median CFU/ml ± SEM.

Figur 2b: S. aureus Proben wurden mit verschiedenen Konzentrationen (0 µM, 1 µM, 10 µM, 100 µM) von Flavin FL-03 für 10 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-03, kein Licht). Die gestrichelte Linie, die mit der Bezeichnung "99.9 % abgetötet" versehen ist, kennzeichnet eine Abnahme der CFU/ml um 3-log₁₀-Stufen; dies entspricht einer Abnahme um 99.9 % ("antibakterielle Wirkung"). N= 3; Median CFU/ml ± SEM.

Figur 2c: E. coli Proben wurden mit verschiedenen Konzentrationen (0 µM, 1 µM, 10 µM, 100 µM) von Flavin FL-03 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-03, kein Licht). Die gestrichelte Linie, die mit der Bezeichnung "99.9 % abgetötet" versehen ist, kennzeichnet eine Abnahme der CFU/ml um 3-log₁₀-Stufen; dies entspricht einer Abnahme um 99.9 % ("antibakterielle Wirkung"). N= 3; Median CFU/ml ± SEM.

Figur 2d: S. aureus Proben wurden mit verschiedenen Konzentrationen (0 µM, 1 µM, 10 µM, 100 µM) von Flavin FL-03 für 30 min inkubiert. Anschließend wurden die Proben entweder mit 50 mW/cm² (210 sec; 10.5 J/cm²) bestrahlt (dunkelgraue Balken) oder nicht bestrahlt (weiße Balken). Nach 24 h wurden die überlebenden Kolonien ausgezählt (CFU/ml). Die schwarze Linie kennzeichnet die Referenz der Dunkelkontrolle (0 µM Flavin FL-03, kein Licht). Die gestrichelte Linie, die mit der Bezeichnung "99.9 % abgetötet" versehen ist, kennzeichnet eine Abnahme der CFU/ml um 3-log₁₀-Stufen; dies entspricht einer Abnahme um 99.9 % ("antibakterielle Wirkung"). N= 3; Median CFU/ml ± SEM.

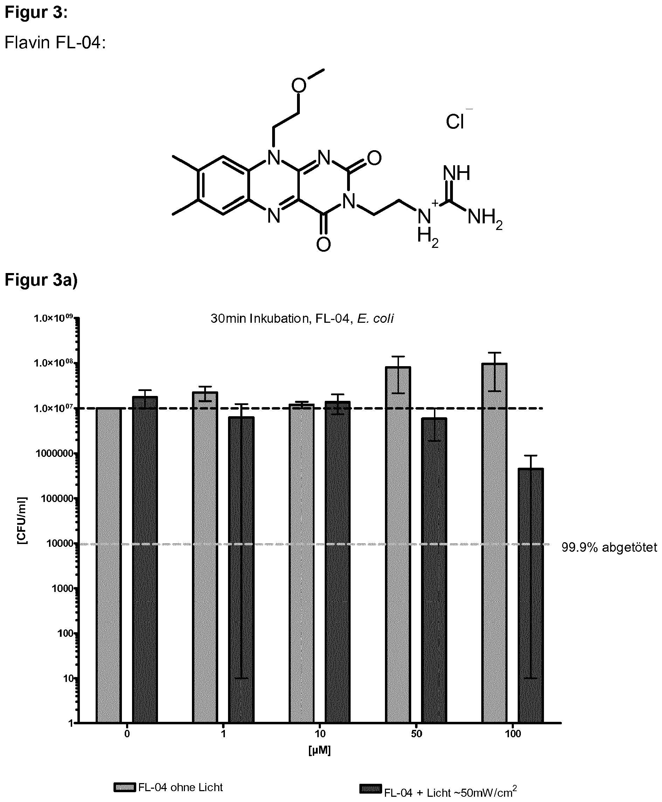

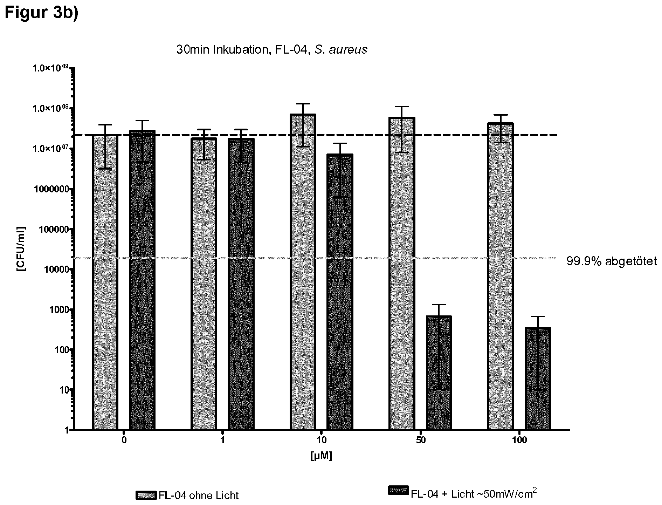

Figur 3 zeigt die Wirkung von Flavin FL-04 (Chlorid der Verbindung mit der Formel (17)) auf E. coli und S. aureus.

Figur 3a: E. coli Proben wurden mit verschiedenen Konzentrationen (0 µM, 1 µM, 10 µM, 50 µM, 100 µM) von Flavin FL-04 für 30 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-04, kein Licht). Die gestrichelte Linie, die mit der Bezeichnung "99.9 % abgetötet" versehen ist, kennzeichnet eine Abnahme der CFU/ml um 3-log₁₀-Stufen; dies entspricht einer Abnahme um 99.9 % ("antibakterielle Wirkung"). N = 3; Median CFU/ml ± SEM.

Figur 3b: S. *aureus* Proben wurden mit verschiedenen Konzentrationen (0 µM, 1 µM, 10 µM, 50 µM, 100 µM) von Flavin FL-04 für 30 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-04, kein Licht). Die gestrichelte Linie, die mit der Bezeichnung "99.9 % abgetötet" versehen ist, kennzeichnet eine Abnahme der CFU/ml um 3-log₁₀-Stufen; dies entspricht einer Abnahme um 99.9 % ("antibakterielle Wirkung"). N = 3; Median CFU/ml ± SEM.

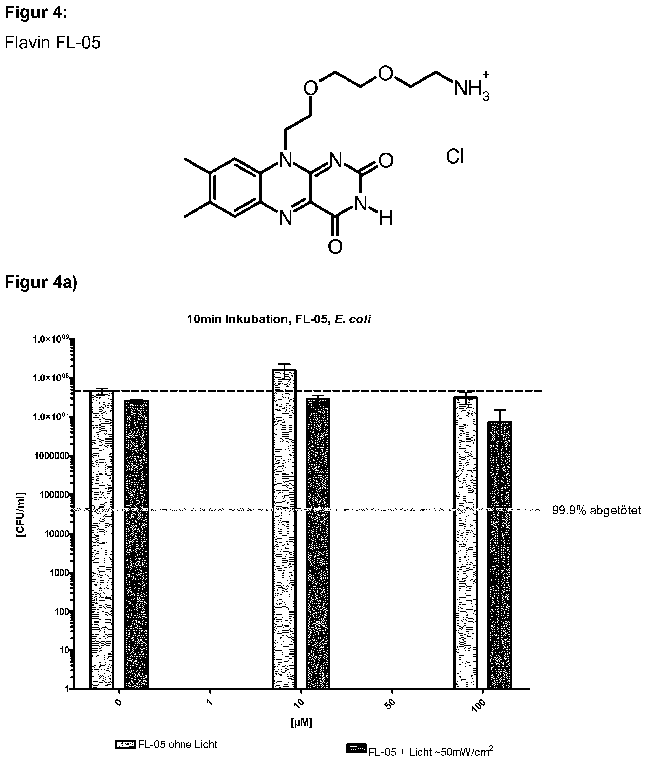

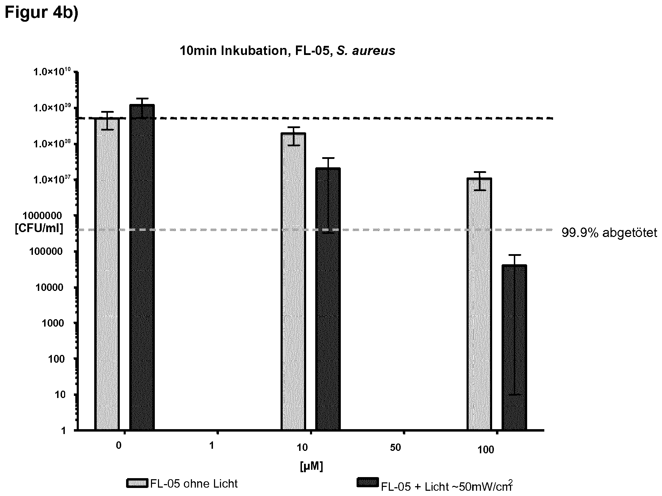

Figur 4 zeigt die Wirkung von Flavin FL-05 (Chlorid der Verbindung mit der Formel (15)) auf E. coli und S. aureus.

Figur 4a: E. coli Proben wurden mit verschiedenen Konzentrationen (0 µM, 10 µM, 100 µM) von Flavin FL-05 für 10 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-05, kein Licht). Die gestrichelte Linie, die mit der Bezeichnung "99.9 % abgetötet" versehen ist, kennzeichnet eine Abnahme der CFU/ml um 3-log₁₀-Stufen; dies entspricht einer Abnahme um 99.9% ("antibakterielle Wirkung"). N = 3; Median CFU/ml ± SEM.

Figur 4b: S. aureus Proben wurden mit verschiedenen Konzentrationen (0 µM, 10 µM, 100 µM) von Flavin FL-05 für 10 min inkubiert. Anschließend wurden die Proben entweder mit 50 mW/cm² (210 sec; 10.5 J/cm²) bestrahlt (dunkelgraue Balken) oder nicht bestrahlt (hellgraue Balken). Nach 24 h wurden die überlebenden Kolonien ausgezählt (CFU/ml). Die schwarze Linie kennzeichnet die Referenz der Dunkelkontrolle (0 µM Flavin FL-05, kein Licht). Die gestrichelte Linie, die mit der Bezeichnung "99.9 % abgetötet" versehen ist, kennzeichnet eine Abnahme der CFU/ml um 3-log₁₀-Stufen; dies entspricht einer Abnahme um 99.9 % ("antibakterielle Wirkung"). N= 3; Median CFU/ml ± SEM.

Wie aus den Figuren 1-4 ersichtlich, hat eine Bestrahlung der verwendeten Mikroorganismen *Staphylococcus aureus* (*S. aureus*) und *Escherichia coli* (*E. coli*) mit einer Lichtdosis von 10.5J/cm² mit blauem Licht (390 nm - 500 nm) in Abwesenheit eines Photosensibilisators (0 µM des jeweiligen Flavins) keinen Einfluss auf die Anzahl der überlebenden Mikroorganismen im Vergleich zur unbelichteten Kontrolle.

Darüber hinaus zeigen die in den Figuren 1-4 dargestellten Ergebnisse, dass die Inkubation (10 min bzw. 30 min) des jeweiligen Photosensibilisators mit den Mikroorganismen ohne nachfolgende Belichtung ebenfalls keinen Einfluss auf die Anzahl der überlebenden Mikroorganismen hat.

Wie aus den Figuren 1 - 4 ersichtlich, erfolgt nach Inkubation (10 min bzw. 30 min) der Mikroorganismen in Abhängigkeit der verwendeten Konzentration der jeweiligen Photosensibilisatoren und nachfolgender Bestrahlung mit einer Lichtdosis von 10.5J/cm² eine Abnahme der KBE/ml und somit eine Inaktivierung von *E. coli* und *S*. *aureus.*

Die Effektivität der Phototoxizität gegenüber Bakterien nach Bestrahlung wurde nach folgenden Richtlinen für Handhygiene im Gesundheitswesen festgelegt (Boyce, J. M., and D. Pittet. 2002. Guideline for Hand Hygiene in Health-Care Settings: recommendations of the Healthcare Infection Control Practices Advisory Committee and the HICPAC/SHEA/APIC/IDSA Hand Hygiene Task Force. Infect Control Hosp Epidemiol 23:S3-40.):

| | |
|---|---|
| - Reduktion der KBE/ml um 1 log₁₀-Stufe | ≙ 90 % Effektivität |
| - Reduktion der KBE/ml um 3 log₁₀-Stufen | ≙ 99,9 % Effektivität |
| - Reduktion der KBE/ml um 5 log₁₀-Stufen | ≙ 99,999 % Effektivität. |

Für eine effektive Inaktivierung kann daher die Abnahme von ≥ 3log₁₀ Stufen angenommen werden, wobei S. *aureus* und *E. coli* als Beispiele für Vertreter aus der Gruppe der Gram-positiven und Gram-negativen Bakterien ausgewählt wurden (siehe Boyce J. M and D. Pittet 2009).

Die benötigte Konzentration, um jeweils eine Reduktion um ≥ 3log₁₀ Stufen zu erzielen, ist in Tabelle 1 dargestellt.

**Tabelle 1: Zusammenfassung photodynamische Inaktivierung**

| **Photosensibilisator** | **Benötigte Konzentration [µM], um eine Reduktion um ≥ 3log₁₀ Stufen zu erzielen (Abnahme um 99.9 %), Bestrahlung mit 10.5 J/cm²** | |
|---|---|---|
| | ***E.coli*** | ***S*. *aureus*** |
| **FL-01** | 10 | 10 |
| **FL-03** | >100 (*) | 10 |
| **FL-04** | >100 (*) | 50 |
| **FL-05** | >100 (*) | 100 |

| | | |
|---|---|---|
| (*) bisher konnte nur eine Reduktion von weniger als 3-log₁₀-Stufen erzielt werden (99 %), bei einer Konzentration von 100 µM und nachfolgender Bestrahlung. | | |

## Patentansprüche

1. Verbindung mit der Formel (1): 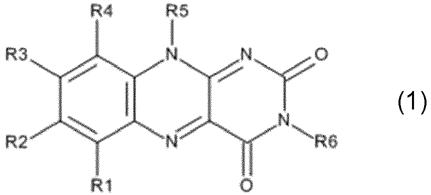 oder ein pharmakologisch verträgliches Salz und/oder Ester und/oder Komplex davon zur Anwendung als Photosensibilisator zur photodynamischen Inaktivierung von Mikroorganismen bei der photodynamischen Therapie einer oberflächlichen Infektionserkrankung,
wobei A) nur 1 Rest R1, R2, R3 oder R4 ein organischer Rest der allgemeinen Formel (2), (3) oder (4):
-(C(D)(E))ₕ-X, -(C(D)(E))ₖ-Aryl-(C(D)(E))ₗ-X, (2) (3) 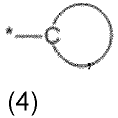
ist,
wobei h eine ganze Zahl von 1 bis 20 und k und I jeweils unabhängig voneinander eine ganze Zahl von 0 bis 6 bedeuten, wobei D und E jeweils unabhängig voneinander Wasserstoff, Halogen, vorzugsweise Chlor, Brom, Iod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol bedeuten und wobei X ein organischer Rest ist, der a) nur ein neutrales, protonierbares Stickstoffatom enthält oder b) nur ein positiv geladenes Stickstoffatom, das kein quartäres Stickstoffatom ist, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
und wobei der Rest mit der Formel (4) ein Heteroaromat, der über ein C-Atom des Heteroaromaten an den Isoalloxazinring gebunden ist und der a) nur ein neutrales, protonierbares Stickstoffatom oder b) nur ein positiv geladenes Stickstoffatom enthält, bedeutet und
wobei die Reste R1, R2, R3 oder R4, die nicht ein organischer Rest der allgemeinen Formel (2), (3) oder (4) sind, jeweils unabhängig voneinander gleich oder verschieden sind und Wasserstoff, Halogen, Hydroxyl, Nitro, Carboxylat, Aldehyd mit 1 bis 20 C-Atomen, Keton mit 2 bis 20 C-Atomen, Thiol, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten, wobei die Alkylreste nichtcyclische Polyolreste der allgemeinen Formel -CH₂(CH(OH))_{g}CH₂OH, wobei g eine ganze Zahl von 1 bis 10, vorzugsweise 1 bis 4, ist, sein können und
wobei jeder der Reste R5 oder R6 jeweils unabhängig voneinander gleich oder verschieden ist und Wasserstoff, Alkyl mit 2 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet, wobei die Alkylreste nichtcyclische Polyolreste der allgemeinen Formel - CH₂(CH(OH))_{g}CH₂OH, wobei g eine ganze Zahl von 1 bis 10, vorzugsweise 1 bis 4, ist, sein können,
oder
wobei B) nur 1 Rest R5 oder R6 ein organischer Rest der allgemeinen Formel (2), (3) oder (4):
-(C(D)(E))ₕ-X, -(C(D) (E))ₖ-Aryl-(C(D)(E))ₗ-X, (2) (3) 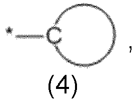
ist,
wobei h eine ganze Zahl von 1 bis 20 und k und l jeweils unabhängig voneinander eine ganze Zahl von 0 bis 6 bedeuten, wobei D und E jeweils unabhängig voneinander Wasserstoff, Halogen, vorzugsweise Chlor, Brom, lod oder Fluor, Hydroxyl, O-R^{(VIII)}, wobei R^{(VIII)} Methyl, Ethyl, n-Propyl, n-Butyl, Phenyl oder Benzyl bedeutet, O-C(=O)-R^{(IX)}, wobei R^{(IX)} Wasserstoff, Methyl, Ethyl, n-Propyl, n-Butyl, Phenyl oder Benzyl bedeutet, oder Thiol bedeuten und wobei X ein organischer Rest ist, der a) nur ein neutrales, protonierbares Stickstoffatom enthält oder b) nur ein positiv geladenes Stickstoffatom, das kein quartäres Stickstoffatom ist, enthält, und Aryl einen substituierten oder unsubstituierten Aromaten mit 5 bis 20 C-Atomen oder einen substituierten oder unsubstituierten Heteroaromaten, der kein Stickstoffatom enthält, mit 4 bis 20 C-Atomen bedeutet
und wobei der Rest mit der Formel (4) ein Heteroaromat, der über ein C-Atom des Heteroaromaten an den Isoalloxazinring gebunden ist und der a) nur ein neutrales, protonierbares Stickstoffatom oder b) nur ein positiv geladenes Stickstoffatom enthält, bedeutet,
und
wobei der Rest R5 oder R6, der nicht ein organischer Rest der allgemeinen Formel (2), (3) oder (4) ist, Wasserstoff, Alkyl mit 1 bis 20 C-Atomen, Alkenyl mit 2 bis 20 C-Atomen, Ether mit 2 bis 20 C-Atomen, Thioether mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeutet, wobei die Alkylreste nichtcyclische Polyolreste der allgemeinen Formel - CH₂(CH(OH))_{g}CH₂OH, wobei g eine ganze Zahl von 1 bis 10, vorzugsweise 1 bis 4, ist, sein können und
wobei die Reste R1 bis R4 jeweils unabhängig voneinander gleich oder verschieden sind und Wasserstoff, Halogen, Hydroxyl, Thiol, Nitro, Carboxylat, Aldehyd mit 1 bis 20 C-Atomen, Keton mit 1 bis 20 C-Atomen, O-Alkyl mit 1 bis 20 C-Atomen, S-Alkyl mit 1 bis 20 C-Atomen, O-Alkenyl mit 2 bis 20 C-Atomen, S-Alkenyl mit 2 bis 20 C-Atomen, O-Aryl mit 5 bis 20 C-Atomen, S-Aryl mit 5 bis 20 C-Atomen, Ether mit 2 bis 20 C-Atomen, Thioether mit 2 bis 20 C-Atomen, Carbonsäureester mit 1 bis 20 C-Atomen, Carbonsäureamid mit 1 bis 20 C-Atomen, Thioester mit 1 bis 20 C-Atomen, Alkyl mit 1 bis 20 C-Atomen, Alkenyl mit 2 bis 20 C-Atomen, Cycloalkyl mit 3 bis 20 C-Atomen, Cycloalkenyl mit 3 bis 20 C-Atomen, Aryl mit 5 bis 20 C-Atomen oder Heteroaryl, das kein Stickstoffatom enthält, mit 4 bis 20 C-Atomen bedeuten, wobei die Alkylreste nichtcyclische Polyolreste der allgemeinen Formel -CH₂(CH(OH))_{g}CH₂OH, wobei g eine ganze Zahl von 1 bis 10, vorzugsweise 1 bis 4, ist, sein können.

2. Verbindung zur Anwendung nach Anspruch 1, wobei X einen Rest der Formel (5): 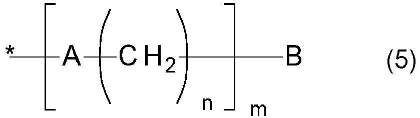 bedeutet und wobei A ein Sauerstoff- oder ein Schwefelatom ist und wobei n eine ganze Zahl von 1 bis 8 und m eine ganze Zahl von 0 bis 100 ist und wobei B ein Rest der Formel (6a), (6b), (7) oder (8): 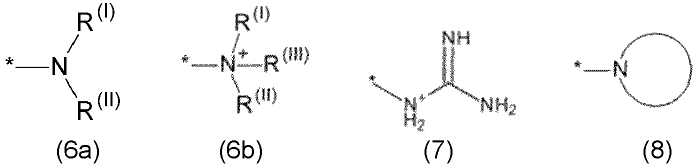 ist und wobei jeder der Reste R^{(I)} und R^{(II)} unabhängig voneinander ausgewählt wird aus Wasserstoff und C1 - C20 Alkyl, das geradkettig oder verzweigt sein kann, und wobei R^{(III)} Wasserstoff ist und wobei der Rest mit der Formel (8) 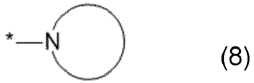 einen substituierten oder unsubstituierten heterocyclischen Rest mit 5 bis 7 Ringatomen, die mindestens ein 1 Kohlenstoffatom und 1 Stickstoffatom sowie optional 1 oder 2 Sauerstoff- oder Schwefelatom umfassen, wobei der heterocyclische Rest gesättigt oder ungesättigt ist, darstellt.

3. Verbindung zur Anwendung nach Anspruch 1, wobei der Rest der Formel (4): 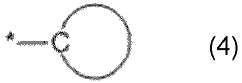 aus der Gruppe, die aus Resten der Formeln (13a), (13b) und (13c): 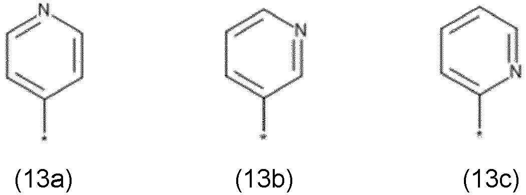 besteht, ausgewählt wird.

4. Verbindung zur Anwendung nach einem der Ansprüche 1 bis 3 wobei die Verbindung mit der Formel (1) aus den Verbindung mit den Formeln (14) bis (17) ausgewählt wird:

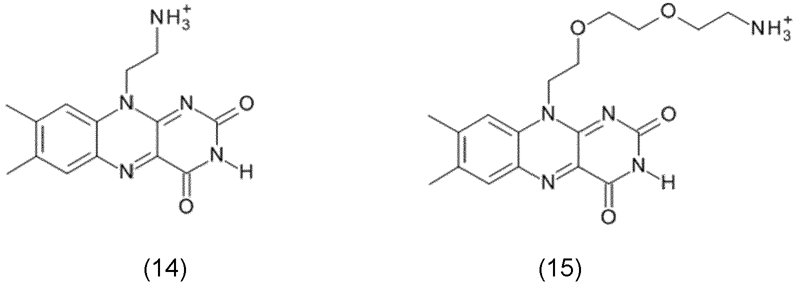

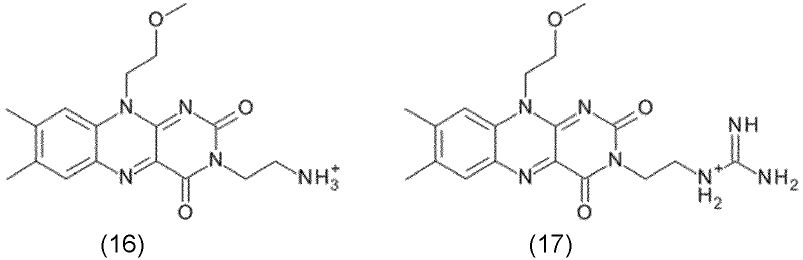

5. Verbindung zur Anwendung nach einem der Ansprüche 1 bis 4, wobei es sich bei den Mikroorganismen um Viren, Archäeen, Bakterien, Bakteriensporen, Pilzen, Pilzsporen, Protozoen, Algen und/oder blutübertragbaren Parasiten handelt.

6. Verbindung zur Anwendung nach einem der Ansprüche 1 bis 5, wobei wenigstens eine Verbindung der Formel (1) oder ein pharmakologisch verträgliches Salz und/oder Ester und/oder Komplex davon zusammen mit wenigstens einem pharmakologisch verträglichen Exzipienten in einer pharmazeutischen Zusammensetzung enthalten sind.

7. Verbindung zur Anwendung nach einem der Ansprüche 1 bis 6, bei der Desinfektion und/oder Dekolonisierung von Körperoberflächen, vorzugsweise Haut und/oder Schleimhaut, eines Säugetiers, vorzugsweise Mensch.

8. Verbindung zur Anwendung nach Anspruch 7, wobei die Körperoberfläche eine Haut- oder Weichteiloberfläche ist.

9. Verbindung zur Anwendung nach einem der Ansprüche 1 bis 8, als Photosensibilisator zur photodynamischen Inaktivierung von Mikroorganismen bei der Prophylaxe und/oder Behandlung von infektiösen Erkrankungen des Ohrs, der oberen Luftwege, der Mundhöhle, des Rachens, des Kehlkopfes, der unteren Luftwege und/oder der Speiseröhre.

10. Verbindung zur Anwendung nach einem der Ansprüche 1 bis 9, als Photosensibilisator zur photodynamischen Inaktivierung von Mikroorganismen in der Mundhöhle bei der Prophylaxe und/oder Behandlung einer Erkrankung des Zahngewebes und/oder des Zahnhalteapparates.

11. Verbindung zur Anwendung nach Anspruch 10, wobei die Erkrankung eine infektiöse Erkrankung des Zahngewebes, vorzugsweise Plaque, Karies oder Pulpitis, und/oder infektiöse Erkrankung des Zahnhalteapparate, vorzugsweise Gingivitis, Paradontitis, Endodontitis oder Periimplantitis, ist.

12. Verbindung zur Anwendung nach einem der Ansprüche 1 bis 6, zur topischen, vorzugsweise nasalen, oralen, analen, vaginalen oder dermalen, Applikation.

## Claims

1. Compound having the formula (1): 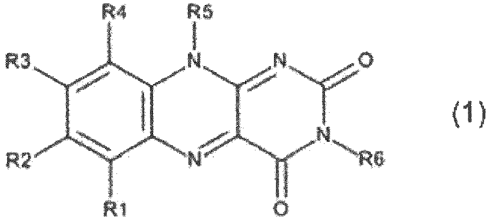 or a pharmacologically acceptable salt and/or ester and/or complex thereof as photosensitizer for photodynamic inactivation of microorganisms in the photodynamic therapy of a superficial infection disorder,
where A) only 1 R1, R2, R3 or R4 radical is an organic radical of the general formula (2), (3) or (4):
-(C(D)(E))ₕ-X, -(C(D)(E))ₖ--Aryl-(C(D)(E))ₗ-X, (2) (3) 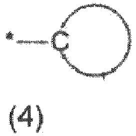
where h is an integer from 1 to 20 and k and 1 are each independently an integer from 0 to 6, where D and E are each independently hydrogen, halogen, preferably chlorine, bromine, iodine or fluorine, hydroxyl, OR^{(VIII)} where R^{(VIII)} is methyl, ethyl, n-propyl, n-butyl, phenyl or benzyl, O-C(=O)-R^{(IX)} where R^{(IX)} is hydrogen, methyl, ethyl, n-propyl, n-butyl, phenyl or benzyl, or thiol, and where X is an organic radical having a) only one uncharged, protonatable nitrogen atom, or b) only one positively charged nitrogen atom which is not a quaternary nitrogen atom, and aryl is a substituted or unsubstituted aromatic system having 5 to 20 carbon atoms or a substituted or unsubstituted heteroaromatic system which does not contain any nitrogen atom and has 4 to 20 carbon atoms,
and where the radical having the formula (4) is a heteroaromatic system which is bonded to the isoalloxazine ring via a carbon atom of the heteroaromatic system and which contains a) only one uncharged, protonatable nitrogen atom or b) only one positively charged nitrogen atom, and
where the R1, R2, R3 or R4 radicals which are not an organic radical of the general formula (2), (3) or (4) are the same or different and are each independently hydrogen, halogen, hydroxyl, nitro, carboxylate, aldehyde having 1 to 20 carbon atoms, ketone having 2 to 20 carbon atoms, thiol, O-alkyl having 1 to 20 carbon atoms, S-alkyl having 1 to 20 carbon atoms, O-alkenyl having 2 to 20 carbon atoms, S-alkenyl having 2 to 20 carbon atoms, O-aryl having 5 to 20 carbon atoms, S-aryl having 5 to 20 carbon atoms, ether having 2 to 20 carbon atoms, thioether having 2 to 20 carbon atoms, carboxylic ester having 1 to 20 carbon atoms, carboxamide having 1 to 20 carbon atoms, thioester having 1 to 20 carbon atoms, alkyl having 1 to 20 carbon atoms, alkenyl having 2 to 20 carbon atoms, cycloalkyl having 3 to 20 carbon atoms, cycloalkenyl having 3 to 20 carbon atoms, aryl having 5 to 20 carbon atoms or heteroaryl which does not contain any nitrogen atom and has 4 to 20 carbon atoms, where the alkyl radicals may be non-cyclic polyol radicals of the general formula -CH₂(CH(OH))_{g}CH₂OH where g is an integer from 1 to 10, preferably 1 to 4,
and
where each of the R5 or R6 radicals is the same or different and is independently hydrogen, alkyl having 2 to 20 carbon atoms, alkenyl having 2 to 20 carbon atoms, ether having 2 to 20 carbon atoms, thioether having 2 to 20 carbon atoms, cycloalkyl having 3 to 20 carbon atoms, cycloalkenyl having 3 to 20 carbon atoms, aryl having 5 to 20 carbon atoms or heteroaryl which does not contain any nitrogen atom and has 4 to 20 carbon atoms, where the alkyl radicals may be non-cyclic polyol radicals of the general formula - CH₂(CH(OH))_{g}CH₂OH where g is an integer from 1 to 10, preferably 1 to 4,
or
where B) only 1 R5 or R6 radical is an organic radical of the general formula (2), (3) or (4):
-(C(D)(E))ₕ-X, -(C(D)(E))ₖ-Aryl-(C(D)(E))ₗ-X, (2) (3) 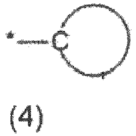
where h is an integer from 1 to 20 and k and 1 are each independently an integer from 0 to 6, where D and E are each independently hydrogen, halogen, preferably chlorine, bromine, iodine or fluorine, hydroxyl, OR^{(VIII)} where R^{(VIII)} is methyl, ethyl, n-propyl, n-butyl, phenyl or benzyl, O-C (=O) -R^{(IX)} where R^{(IX)} is hydrogen, methyl, ethyl, n-propyl, n-butyl, phenyl or benzyl, or thiol, and where X is an organic radical having a) only one uncharged, protonatable nitrogen atom, or b) only one positively charged nitrogen atom which is not a quaternary nitrogen atom, and aryl is a substituted or unsubstituted aromatic system having 5 to 20 carbon atoms or a substituted or unsubstituted heteroaromatic system which does not contain any nitrogen atom and has 4 to 20 carbon atoms,
and where the radical having the formula (4) is a heteroaromatic system which is bonded to the isoalloxazine ring via a carbon atom of the heteroaromatic system and which contains a) only one uncharged, protonatable nitrogen atom or b) only one positively charged nitrogen atom,
and
where the R5 or R6 radical which is not an organic radical of the general formula (2), (3) or (4) is hydrogen, alkyl having 1 to 20 carbon atoms, alkenyl having 2 to 20 carbon atoms, ether having 2 to 20 carbon atoms, thioether having 2 to 20 carbon atoms, cycloalkyl having 3 to 20 carbon atoms, cycloalkenyl having 3 to 20 carbon atoms, aryl having 5 to 20 carbon atoms or heteroaryl which does not contain any nitrogen atom and has 4 to 20 carbon atoms, where the alkyl radicals may be non-cyclic polyol radicals of the general formula -CH₂(CH(OH))_{g}CH₂OH where g is an integer from 1 to 10, preferably 1 to 4,
and
where the R1 to R4 radicals are the same or different and are each independently hydrogen, halogen, hydroxyl, thiol, nitro, carboxylate, aldehyde having 1 to 20 carbon atoms, ketone having 1 to 20 carbon atoms, O-alkyl having 1 to 20 carbon atoms, S-alkyl having 1 to 20 carbon atoms, O-alkenyl having 2 to 20 carbon atoms, S-alkenyl having 2 to 20 carbon atoms, O-aryl having 5 to 20 carbon atoms, S-aryl having 5 to 20 carbon atoms, ether having 2 to 20 carbon atoms, thioether having 2 to 20 carbon atoms, carboxylic ester having 1 to 20 carbon atoms, carboxamide having 1 to 20 carbon atoms, thioester having 1 to 20 carbon atoms, alkyl having 1 to 20 carbon atoms, alkenyl having 2 to 20 carbon atoms, cycloalkyl having 3 to 20 carbon atoms, cycloalkenyl having 3 to 20 carbon atoms, aryl having 5 to 20 carbon atoms or heteroaryl which does not have any nitrogen atom and has 4 to 20 carbon atoms, where the alkyl radicals may be non-cyclic polyol radicals of the general formula - CH₂(CH(OH))_{g}CH₂OH where g is an integer from 1 to 10, preferably 1 to 4.

2. Compound for use according to Claim 1, where X is a radical of the formula (5): 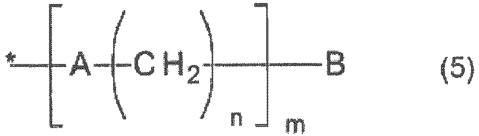 and where A is an oxygen or sulfur atom and where n is an integer from 1 to 8 and m is an integer from 0 to 100, and where B is a radical of the formula (6a), (6b), (7) or (8): 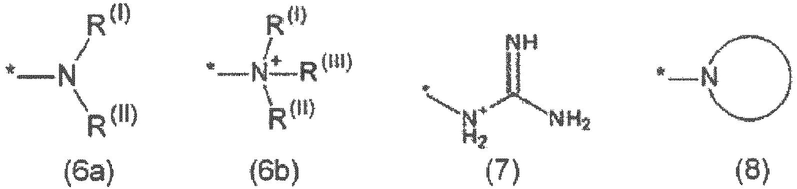 and where each of the R^{(I)} and R^{(II)} radicals is independently selected from hydrogen and C1 - C20 alkyl which may be straight-chain or branched, and where R^{(III)} is hydrogen and where the radical having the formula (8) 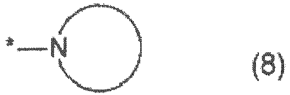 is a substituted or unsubstituted heterocyclic radical having 5 to 7 ring atoms including at least one 1 carbon atom and 1 nitrogen atom and optionally 1 or 2 oxygen or sulfur atoms, where the heterocyclic radical is saturated or unsaturated.

3. Compound for use according to Claim 1, where the radical of the formula (4): 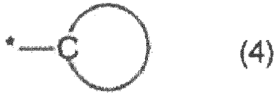 is selected from the group consisting of radicals of the formulae (13a), (13b) and (13c):

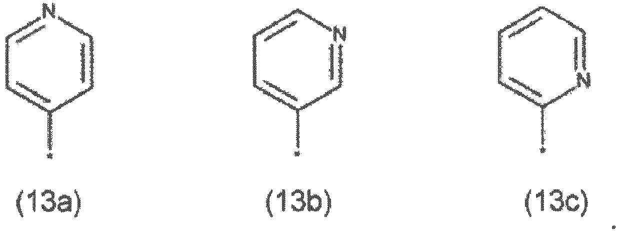

4. Compound for use according to any of Claims 1 to 3, where the compound having the formula (1) is selected from the compounds having the formulae (14) to (17) :

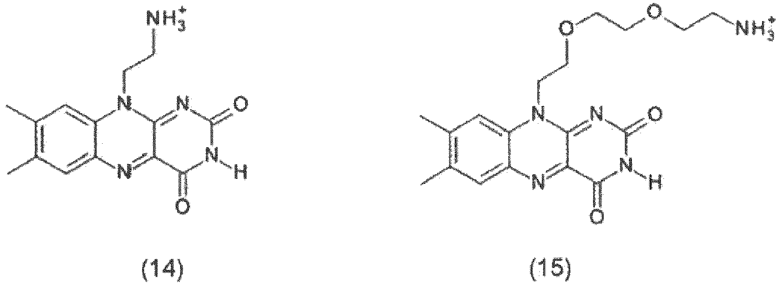

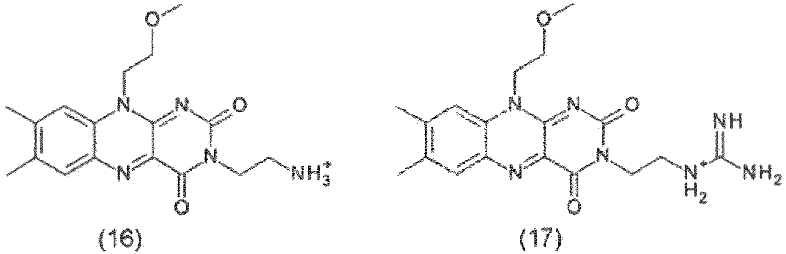

5. Compound for use according to any of Claims 1 to 4, where the microorganisms are viruses, archaea, bacteria, bacterial spores, fungi, fungal spores, protozoa, algae and/or blood-transmissible parasites.

6. Compound for use according to any of Claims 1 to 5, where at least one compound of the formula (1) or a pharmacologically acceptable salt and/or ester and/or complex thereof is present in a pharmaceutical composition together with at least one pharmacologically acceptable excipient.

7. Compound for use according to any of Claims 1 to 6, in the disinfection and/or decolonization of surfaces of the body, preferably the skin and/or mucous membrane, of a mammal, preferably human.

8. Compound for use according to Claim 7, wherein the surface of the body is a skin surface or soft tissue surface.

9. Compound for use according to any of Claims 1 to 8, as photosensitizer for photodynamic inactivation of microorganisms in the prophylaxis and/or treatment of infectious diseases of the ear, of the upper respiratory pathway, of the oral cavity, of the throat, of the larynx, of the lower respiratory pathway and/or of the oesophagus.

10. Compound for use according to any of Claims 1 to 9, as photosensitizer for photodynamic inactivation of microorganisms in the prophylaxis and/or treatment of a disorder of the dental tissue and/or periodontium.

11. Compound for use according to Claim 10, where the disorder is an infectious disorder of the dental tissue, preferably plaque, caries or pulpitis, and/or infectious disorder of the periodontium, preferably gingivitis, paradontitis, endodontitis or periimplantitis.

12. Compound for use according to any of Claims 1 to 6, for topical, preferably nasal, oral, anal, vaginal or dermal, administration.

## Revendications

1. Composé de formule (1) : 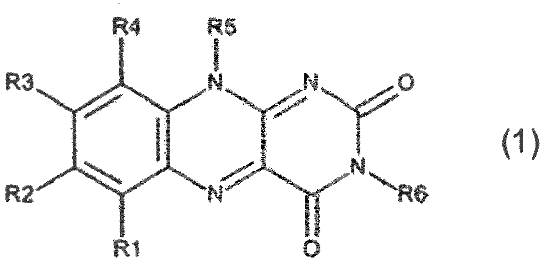 ou un sel et/ou ester et/ou complexe pharmacologiquement acceptable de celui-ci, pour une utilisation en tant que photosensibilisateur pour l'inactivation photodynamique de micro-organismes, lors du traitement photodynamique d'une maladie infectieuse superficielle,
dans laquelle A) seul 1 radical R1, R2, R3 ou R4 est un radical organique de formule générale (2), (3) ou (4) :
-(C(D)(E))ₕ-X, -(C(D)(E))ₖ-Aryl-(C(DXE))ₗ-X, (2) (3) 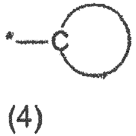
dans laquelle h est un nombre entier de 1 à 20 et k et l représentent chacun indépendamment de l'autre un nombre entier de 0 à 6, D et E représentent chacun indépendamment de l'autre un atome d'hydrogène, un atome d'halogène, de préférence le chlore, le brome, l'iode ou le fluor, un groupe hydroxyle, O-R^{(VIII)}, où R^{(VIII)} représente le groupe méthyle, éthyle, n-propyle, n-butyle, phényle ou benzyle, O-C(=O)-R^{(IX)}, R^{(IX)} représentant un atome d'hydrogène, le groupe méthyle, éthyle, n-propyle, n-butyle, phényle ou benzyle, ou un groupe thiol, et X représente un radical organique, qui a) ne contient qu'un atome d'azote protonable neutre, ou b) ne contient qu'un atome d'azote positivement chargé, qui n'est pas un atome d'azote quaternaire, et aryle représente un radical aromatique substitué ou non substitué ayant 5 à 20 atomes de carbone ou un radical hétéroaromatique substitué ou non substitué, qui ne contient aucun atome d'azote, ayant 4 à 20 atomes de carbone,
et dans laquelle le radical de formule (4) est un radical hétéroaromatique, qui est relié par l'intermédiaire d'un atome de carbone du radical hétéroaromatique au noyau isoalloxazine, et qui a) ne contient qu'un atome d'azote protonable neutre, ou b) ne contient qu'un atome d'azote positivement chargé, et
dans laquelle les radicaux R1, R2, R3 ou R4, qui ne représentent pas des radicaux organiques de formule générale (2), (3) ou (4), sont chacun indépendamment des autres identiques ou différents et représentent un atome d'hydrogène, un halogène, un groupe hydroxyle, nitro, carboxylate, aldéhyde ayant 1 à 20 atomes de carbone, cétone ayant 2 à 20 atomes de carbone, thiol, O-alkyle ayant 1 à 20 atomes de carbone, S-alkyle ayant 1 à 20 atomes de carbone, O-alcényle ayant 2 à 20 atomes de carbone, S-alcényle ayant 2 à 20 atomes de carbone, O-aryle ayant 5 à 20 atomes de carbone, S-aryle ayant 5 à 20 atomes de carbone, éther ayant 2 à 20 atomes de carbone, thioéther ayant 2 à 20 atomes de carbone, ester d'acide carboxylique ayant 1 à 20 atomes de carbone, carboxamide ayant 1 à 20 atomes de carbone, thioester ayant 1 à 20 atomes de carbone, alkyle ayant 1 à 20 atomes de carbone, alcényle ayant 2 à 20 atomes de carbone, cycloalkyle ayant 3 à 20 atomes de carbone, cycloalcényle ayant 3 à 20 atomes de carbone, aryle ayant 5 à 20 atomes de carbone ou hétéroaryle, qui ne contient aucun atome d'azote, ayant 4 à 20 atomes de carbone, les radicaux alkyle pouvant être des radicaux polyol non cycliques de formule générale - CH₂(CH(OH))_{g}CH₂OH, dans laquelle g est un nombre entier de 1 à 10, de préférence de 1 à 4,
et
dans laquelle chacun des radicaux R5 ou R6 est, indépendamment de l'autre, identique ou différent, et représente un atome d'hydrogène, un groupe alkyle ayant 2 à 20 atomes de carbone, alcényle ayant 2 à 20 atomes de carbone, éther ayant 2 à 20 atomes de carbone, thioéther ayant 2 à 20 atomes de carbone, cycloalkyle ayant 3 à 20 atomes de carbone, cycloalcényle ayant 3 à 20 atomes de carbone, aryle ayant 5 à 20 atomes de carbone ou hétéroaryle, qui ne contient aucun atome d'azote, ayant 4 à 20 atomes de carbone, les groupes alkyle étant des groupes polyol non cycliques de formule générale - CH₂(CH(OH))_{g}CH₂OH, dans laquelle g est un nombre entier de 1 à 10, de préférence de 1 à 4,
ou
dans laquelle B) seul 1 un radical R5 ou R6 est un radical de formule générale (2), (3) ou (4) :
-(C(D)(E))ₕ-X, -(C(D)(E))ₖ-Aryl-(C(D)(E))ₗ-X, (2) (3) 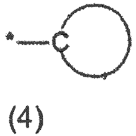
dans laquelle h est un nombre entier de 1 à 20 et k et I représentent chacun indépendamment de l'autre un nombre entier de 0 à 6, D et E représentent chacun indépendamment de l'autre un atome d'hydrogène, un atome d'halogène, de préférence le chlore, le brome, l'iode ou le fluor, un groupe hydroxyle, O-R^{(VIII)}, où R^{(VIII)} représente le groupe méthyle, éthyle, n-propyle, n-butyle, phényle ou benzyle, O-C(=O)-R^{(IX)}, R^{(IX)} représentant un atome d'hydrogène, le groupe méthyle, éthyle, n-propyle, n-butyle, phényle ou benzyle, ou un groupe thiol, et X représente un radical organique, qui a) ne contient qu'un atome d'azote protonable neutre, ou b) ne contient qu'un atome d'azote positivement chargé, qui n'est pas un atome d'azote quaternaire, et aryle représente un radical aromatique substitué ou non substitué ayant 5 à 20 atomes de carbone ou un radical hétéroaromatique substitué ou non substitué, qui ne contient aucun atome d'azote, ayant 4 à 20 atomes de carbone,
et dans lequel le radical de formule (4) est un radical hétéroaromatique, qui est relié par l'intermédiaire d'un atome de carbone du radical hétéroaromatique au noyau isoalloxazine, et qui a) ne contient qu'un atome d'azote protonable neutre, ou b) ne contient qu'un atome d'azote positivement chargé, et
dans laquelle le radical R5 ou R6, qui n'est pas un radical organique de formule générale (2), (3) ou (4), représente un atome d'hydrogène, un groupe alkyle ayant 1 à 20 atomes de carbone, alcényle ayant 2 à 20 atomes de carbone, éther ayant 2 à 20 atomes de carbone, thioéther ayant 2 à 20 atomes de carbone, cycloalkyle ayant 3 à 20 atomes de carbone, cycloalcényle ayant 3 à 20 atomes de carbone, aryle ayant 5 à 20 atomes de carbone ou hétéroaryle, qui ne contient aucun atome d'azote, ayant 4 à 20 atomes de carbone, les radicaux alkyle pouvant être des radicaux polyol non cycliques de formule générale - CH₂(CH(OH))_{g}CH₂OH, dans laquelle g est un nombre entier de 1 à 10, de préférence de 1 à 4, et
dans laquelle les radicaux R1 à R4 sont chacun indépendamment des autres identiques ou différents et représentent chacun un atome d'hydrogène, un halogène, un groupe hydroxyle, thiol, nitro, carboxylate, aldéhyde ayant 1 à 20 atomes de carbone, cétone ayant 1 à 20 atomes de carbone, O-alkyle ayant 1 à 20 atomes de carbone, S-alkyle ayant 1 à 20 atomes de carbone, O-alcényle ayant 2 à 20 atomes de carbone, S-alcényle ayant 2 à 20 atomes de carbone, O-aryle ayant 5 à 20 atomes de carbone, S-aryle ayant 5 à 20 atomes de carbone, éther ayant 2 à 20 atomes de carbone, thioéther ayant 2 à 20 atomes de carbone, ester d'acide carboxylique ayant 1 à 20 atomes de carbone, carboxamide ayant 1 à 20 atomes de carbone, thioester ayant 1 à 20 atomes de carbone, alkyle ayant 1 à 20 atomes de carbone, alcényle ayant 2 à 20 atomes de carbone, cycloalkyle ayant 3 à 20 atomes de carbone, cycloalcényle ayant 3 à 20 atomes de carbone, aryle ayant 5 à 20 atomes de carbone ou hétéroaryle, qui ne contient aucun atome d'azote, ayant 4 à 20 atomes de carbone, les radicaux alkyle pouvant être des radicaux polyol non cycliques de formule générale - CH₂(CH(OH))_{g}CH₂OH, dans laquelle g est un nombre entier de 1 à 10, de préférence de 1 à 4.

2. Composé pour une utilisation selon la revendication 1, dans lequel X est un radical de formule (5) : 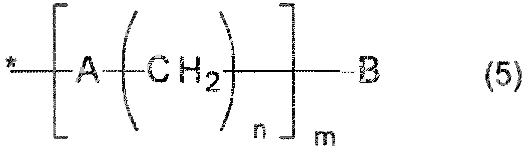 et dans lequel A représente un atome d'oxygène ou de soufre, et n est un nombre entier de 1 à 8 et m est un nombre entier de 0 à 100, et B représente un radical de formule (6a), (6b), (7) ou (8) : 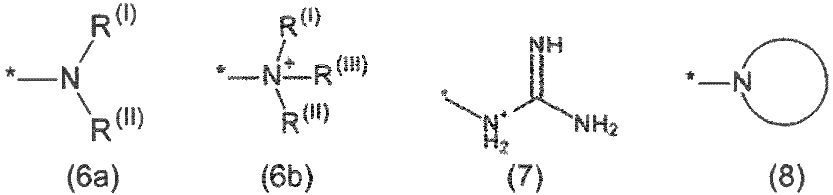 et dans lequel chacun des radicaux R^{(I)} et R^{(II)} est indépendamment de l'autre choisi parmi l'atome d'hydrogène et un radical alkyle en C1-C20, qui peut être à chaîne droite ou ramifiée, et R^{(III)} représente un atome d'hydrogène, et le radical de formule (8) 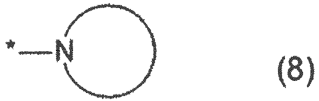 représente un radical hétérocyclique substitué ou non substitué ayant 5 à 7 atomes nucléaires, qui comprennent au moins 1 atome de carbone et 1 atome d'azote, ainsi qu'en option 1 ou 2 atomes d'oxygène ou de soufre, le radical hétérocyclique étant saturé ou insaturé.

3. Composé pour une utilisation selon la revendication 1, dans lequel le radical de formule (4) : 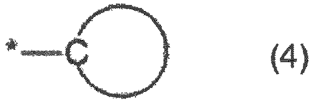 est choisi dans le groupe consistant en les radicaux de formules (13a), (13b) et (13c) :

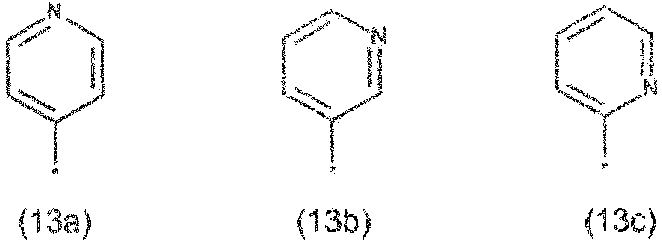

4. Composé pour une utilisation selon l'une des revendications 1 à 3, dans lequel le composé de formule (1) est choisi parmi les composés ayant les formules (14) à (17) :

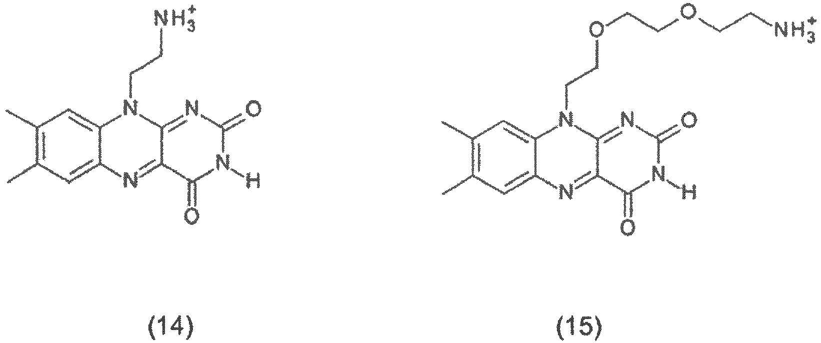

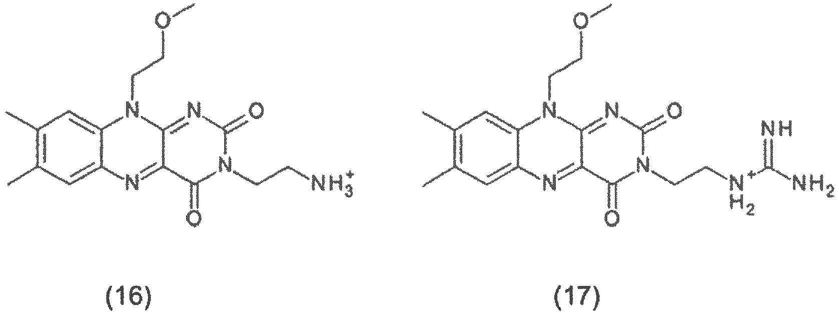

5. Composé pour une utilisation selon l'une des revendications 1 à 4, dans lequel, pour ce qui concerne les micro-organismes, il s'agit de virus, d'archées, de bactéries, de spores bactériennes, de champignons, de spores fongiques, de protozoaires, d'algues et/ou de parasites transmissibles par le sang.

6. Composé pour une utilisation selon l'une des revendications 1 à 5, dans lequel au moins un composé de Formule (1) ou un sel et/ou un ester et/ou un complexe pharmacologiquement acceptable de celui-ci est contenu, conjointement avec au moins un excipient pharmacologiquement acceptable, dans une composition pharmaceutique.

7. Composé pour une utilisation selon l'une des revendications 1 à 6 pour la désinfection et/ou la décolonisation des surfaces du corps, de préférence la peau et/ou la muqueuse, d'un mammifère, de préférence d'un être humain.

8. Composé pour une utilisation selon la revendication 7, dans lequel la surface du corps est une surface de peau ou de partie molle.

9. Composé pour une utilisation selon l'une des revendications 1 à 8 en tant que photosensibilisateur pour l'inactivation photodynamique de micro-organismes lors de la prophylaxie et/ou du traitement de maladies infectieuse de l'oreille, des voies respiratoires supérieures, de la cavité buccale, du pharynx, du larynx, des voies respiratoires inférieures et/ou de l'œsophage.

10. Composé pour une utilisation selon l'une des revendications 1 à 8 en tant que photosensibilisateur pour l'inactivation photodynamique de micro-organismes dans la cavité buccale lors de la prophylaxie et/ou du traitement d'une maladie du tissu dentaire et/ou du parodonte.

11. Composé pour une utilisation selon la revendication 10, dans lequel la maladie est une maladie infectieuse du tissu dentaire, de préférence la plaque dentaire, une carie ou une pulpite et/ou une maladie infectieuse du parodonte, de préférence une gingivite, une parodontite, une endodontite ou une péri-implantite.

12. Composé pour une utilisation selon l'une des revendications 1 à 6 pour une application topique, de préférence nasale, orale, anale, vaginale ou cutanée.
